# EUROPEAN PATENT APPLICATION

(11) **EP 4 446 339 A1**
(43) Date of publication of application: **16.10.2024**
(21) Application number: 22902874.1
(22) Date of filing: 15.08.2022
(51) Int. Cl.: C07K 16/10, C12N 15/13, G01N 33/569, A61K 39/42, A61P 31/14

(54) **ANTIBODY FOR RECOGNIZING RSV PRE-F PROTEIN AND USE THEREOF**

(30) Priority: 06.12.2021 CN 202111477846
(71) Applicant: Xiamen University, Xiamen, Fujian 361005 (CN)
(72) Inventor: ZHENG, Zizheng, Xiamen, Fujian 361005 (CN); SUN, Yongpeng, Xiamen, Fujian 361005 (CN); LEI, Siyu, Xiamen, Fujian 361005 (CN); QIANG, Hongsheng, Xiamen, Fujian 361005 (CN); CHEN, Li, Xiamen, Fujian 361005 (CN); XIA, Ningshao, Xiamen, Fujian 361005 (CN)
(74) Representative: Meissner Bolte Partnerschaft mbB
(86) International application number: PCT/CN2022/112500
(87) International publication number: WO 2023/103440

(57) **Abstract**

The present invention relates to the field of immunology and molecular virology, and in particular relates to the field of the prevention and treatment of RSV. Specifically, provided are a monoclonal antibody capable of specifically binding to a new epitope between an Ø epitope and a V epitope on a pre-F protein or a new epitope between an II epitope and a V epitope, and the use thereof in the detection, prevention and/or treatment of RSV infection and/or diseases caused by the infections.

## Description

### Technical Field

The present application relates to the fields of immunology and molecular virology, especially the fields of prevention and treatment of RSV. Specifically, the present application provides monoclonal antibodies that can specifically bind to a new epitope located between the antigenic site Ø and site V or a new epitope located between the antigenic site II and site V on the pre-F protein, and use thereof for the detection, prevention and/or treatment of RSV infection and/or diseases caused by the infection.

### Background Art

Human respiratory syncytial virus (RSV) is the most common pathogen causing acute lower respiratory tract infections in children under five years old, and is also the leading cause of hospitalization for lower respiratory tract infections in infants and young children. Almost all infants and young children under 2 years old have been infected with RSV, and infants and young children under 6 months old are the main group of people hospitalized due to RSV infection, accounting for about 50%. There are more than 30 million cases of lower respiratory tract infections caused by RSV infection globally every year, including more than 3 million hospitalizations and 66,000 to 239,000 deaths (Ting Shi, David A McAllister, et al., Lancet, 390 (2017) 946-958; Geoghegan S, Erviti A, et al., Am J Respir Crit Care Med 195 (2017) 96-103). Compared with other respiratory virus infections, children infected with RSV have a higher disease burden; in particular, infants and young children under 6 months old have a higher disease burden than children 6-24 months old, including higher ICU admission rates, hospitalization costs, and longer hospital stays. RSV infection increases the risk of severe pediatric pneumonia by 14 times (The Pneumonia Etiology Research for Child Health (PERCH) Study Group, Lancet, 394 (2019) 757-779). The RSV infection rate in premature infants, those with bronchial and pulmonary hypoplasia, congenital heart disease, and immunodeficiency is as high as 50% to 70% (A.C. Cooper, N.C. Banasiak, et al., Pediatr Nurs, 29 (2003) 452 -456). In addition, the elderly are also susceptible to RSV, with more than 12,000 deaths caused by RSV infection every year. RSV is also the main pathogen of lower respiratory tract infection in Chinese children. In China, approximately 215,000 to 500,000 infants and young children are hospitalized due to RSV infection every year (Li Y, Johnson EK, et al., Lancet Respir Med, 2021, 9 (2): 175-185).

There is currently no safe and effective RSV vaccine on the market. Only one neutralizing antibody (Palivizumab, trade name: Synagis) that recognizes RSV fusion glycoprotein has been approved by FDA of the US for the prevention of severe lower respiratory tract infection caused by RSV in patients such as premature infants, high-risk infants and young children with chronic lung diseases, bronchial and pulmonary dysplasia, and congenital heart disease. However, due to the insufficient neutralizing potency, high production cost, and high price, Synagis is only used for "infants and young children with high risk of infection" and has not been widely used. In addition, Sanofi Pasteur and AstraZeneca jointly developed a new generation of RSV preventive drug "Nirsevimab" based on the human antibody D25. Nirsevimab is a preventive monoclonal antibody with extended half-life, and one single dose of intramuscular injection of Nirsevimab can provide long-lasting protection for up to 5 months in infants and young children, allowing infants and young children to safely survive the entire RSV epidemic season. The results of the clinical phase IIb trial showed that compared with the control group, Nirsevimab reduced the rates of medical consultation and hospitalization by 70.1% and 78.4%, respectively (M Pamela Griffin, Yuan Yuan, et al., N Engl J Med, 383(2020) 415-425). At present, Nirsevimab has been designated as a "breakthrough therapy" by FDA of the US. In January 2021, the monoclonal antibody Nirsevimab was listed as a "breakthrough therapy drug" in China. Currently, there are no RSV-specific preventive or therapeutic drugs on the market in China.

RSV is a single-stranded negative-strand non-segmented RNA virus belonging to the family *Pneumoniavirus* of *Adhesionviridae.* It has 15,222 nucleotides, contains 10 genes, and encodes 11 proteins. Among them, the fusion (F) protein, which is a type I transmembrane protein, has a full length of 574 amino acids and is currently the main target protein for RSV vaccine and antibody development. RSV F protein exists in two conformations: pre-fusion (pre-F) and post-fusion (post-F). Pre-F is a high-energy, metastable pre-fusion conformation that mediates the fusion of viral envelope and cell membrane; after the fusion, pre-F allosterically transforms into the highly stable post-fusion conformation post-F. Compared with post-F protein, pre-F protein has more neutralizing epitopes with higher activity, which can induce the production of antibodies with high neutralizing activity.

In summary, RSV has caused serious harm to children and heavy health and medical burdens around the world. In China, it urgently needs to develop antibodies with independent intellectual property rights that possess broad-spectrum and high-neutralizing activity for RSV prevention and/or treatment, and the developed antibodies can be used for the prevention and/or treatment of RSV infection in children.

### Contents of the present application

Currently, six neutralizing epitopes (sites Ø, I, II, III, IV and V) have been discovered on pre-F and post-F. Among them, the sites Ø and V are epitopes that are specifically present on pre-F with high activity, the sites I, II, III and IV are present on both pre-F and post-F. The site Ø is specifically located at the membrane-distal apex of the pre-F trimer. This epitope comprises the α4 helix (residues 196-209) of the F1 subunit and part region (residues 62-69) of F2; and both regions undergo dramatic changes during the allosteric process from prefusion to postfusion. The antibodies that recognize this epitope include D25, 5C4 and AM22. The three antibodies are all pre-F-specific antibodies with potent neutralizing activity, and their neutralizing potencies are almost 100 times higher than that of Palivizumab. The site I exists on both pre-F and post-F, and is a linear epitope consisting of residues 387-392 located between α8 helix and β14; and the antibody that recognizes this epitope is 131-2a, while this antibody has poor neutralizing ability. The site II exists on both pre-F and post-F, and consists of α6 helix, α7 helix and the loop between them (residues 255-276). Representative antibodies that recognize this epitope include mouse antibody 1129, humanized Palivizumab, and the second-generation antibody Motavizumab derived from palivizumab; and these antibodies exhibit moderate neutralizing abilities. The site III also exists on both pre-F and post-F. The representative antibody that recognizes this epitope is MPE8, which prefers to bind to the protein in the pre-F conformation. MPE8 has broad-spectrum neutralizing activity and can neutralize four kinds of viruses, hRSV, BRSV, hMPV and PVM. The site IV also exists on both pre-F and post-F, and has a linear structure composed of residues 422-438; and the representative antibody that recognizes this epitope is 101F, which exhibits moderate neutralizing ability. The site V specifically exists on the pre-F conformational protein, and is an epitope composed of residues 146-194 and 287-300 on the protein trimer; and the antibody AM14 that recognizes this epitope can only bind to the two monomer binding regions of pre-F and has a high neutralizing ability.

After extensive research, the inventors of the present application unexpectedly developed a monoclonal antibody that can specifically bind to a new epitope located between the antigenic site Ø and site V or a new epitope located between the antigenic site II and site V on the pre-F protein. The antibody of the present application can specifically recognize the pre-F conformation, effectively neutralize RSV, and block or inhibit the fusion of RSV and cells. The present application thus provides the following aspects.

### Antibody or antigen-binding fragment thereof

### 5B11

In a first aspect, the present application provides an antibody or antigen-binding fragment thereof that specifically binds to an epitope of respiratory syncytial virus (RSV) pre-F protein, wherein the epitope comprises at least 6 amino acid residues (e.g., non-consecutive amino acid residues) within the amino acid residues at positions 160-182 and comprises the amino acid residues at positions 294-295 of the pre-F protein.

In certain embodiments, the epitope comprises at least the amino acid residues at positions 161, 165, 166, 169, 180, 182, 294, and 295.

In certain embodiments, the epitope further comprises the amino acid residue at position 196.

In certain embodiments, the epitope further comprises the amino acid residues at positions 162 and 184.

In certain embodiments, the epitope comprises the amino acid residues at positions 161-182 (e.g., the amino acid residues at positions 161-184) and the amino acid residues at positions 294-295.

In certain embodiments, the epitope is a conformational epitope.

In certain embodiments, the position of amino acid residue is determined according to SEQ ID NO: 1.

In certain embodiments, the epitope comprises at least E161, N165, K166, S 169, S 180, S182, E294, and E295; in certain embodiments, the epitope further comprises K196; in certain embodiments, the epitope also comprises G162 and G184.

In a second aspect, the present application provides an antibody or antigen-binding fragment thereof, comprising:
(a) a heavy chain variable region (VH) comprising the following three CDRs: a VH CDR1 comprising the sequence as set forth in SEQ ID NO: 5 or variant thereof, a VH CDR2 comprising the sequence as set forth in SEQ ID NO: 6 or variant thereof, a VH CDR3 comprising the sequence as set forth in SEQ ID NO: 7 or variant thereof; and/or,
(b) a light chain variable region (VL) comprising the following three CDRs: a VL CDR1 comprising the sequence as set forth in SEQ ID NO: 8 or variant thereof, a VL CDR2 comprising the sequence as set forth in SEQ ID NO: 9 or variant thereof, a VL CDR3 comprising the sequence as set forth in SEQ ID NO: 10 or variant thereof;
wherein, the variant has a sequence identity of at least 70%, at least 80%, at least 85%, at least 90%, at least 91%, at least 92%, at least 93%, at least 94%, at least 95% %, at least 96%, at least 97%, at least 98%, at least 99%, or 100% as compared to the sequence from which it is derived, or the variant has a substitution, deletion, or addition of one or several amino acids (e.g., a substitution, deletion or addition of 1, 2 or 3 amino acids) as compared to the sequence from which it is derived. In certain embodiments, the substitution is a conservative substitution.

In certain embodiments, the antibody or antigen-binding fragment thereof is capable of specifically binding RSV pre-F protein.

In certain embodiments, the antibody or antigen-binding fragment thereof competes with the antibody or antigen-binding fragment thereof of the first aspect for binding to RSV pre-F protein. In certain embodiments, the competitive binding refers to being able to block at least 50%, preferably at least 60%, preferably at least 70%, preferably at least 80%, preferably at least 90%, preferably at least 95% or preferably at least 99% of the binding of the antibody or antigen-binding fragment thereof of the first aspect to the RSV pre-F protein. The competitive binding can be determined by a competitive binding assay.

Competitive binding assays are well known to those skilled in the art. Competitive binding assays are immunological assays in which an unknown substance is detected and quantified through the ability of the unknown substance to inhibit the binding of a labeled known antigen to an antibody to which it specifically binds, and are also called competitive inhibition assays. An exemplary method comprises: an antigen is pre-coated on a microwell plate, then an unlabeled antibody to be tested that is serially diluted and a labeled known monoclonal antibody (i.e., the antibody or antigen-binding fragment thereof as described in the first aspect) with a specific concentration are added to the above-mentioned pre-coated microwell plate for incubation, and then after washing, the amount of the known antibody bound to the plate in the presence of the antibody to be tested at different dilutions is measured. The greater the ability of the antibody to be tested to compete with the known antibody for binding to the antigen, the weaker the ability of the known antibody to bind to the antigen, and the less the known antibody to bind to the plate. Usually, the antigen is pre-coated on a 96-well microplate, and the ability of the monoclonal antibody to be tested to block the labeled known monoclonal antibody is determined by a radioactive immunoassay, an enzyme immunoassay such as ELISA, or a fluorescent immunoassay.

In certain embodiments, the epitope recognized by the antibody or antigen-binding fragment thereof is the same as, or overlaps in space with, the epitope recognized by the antibody or antigen-binding fragment thereof described in the first aspect, such that the antibody or antigen-binding fragment thereof can block the binding of the antibody or antigen-binding fragment thereof of the first aspect to the RSV pre-F protein. In certain embodiments, the antibody or antigen-binding fragment thereof binds to the same epitope of the RSV pre-F protein as the antibody or antigen-binding fragment thereof of the first aspect.

In certain embodiments:
(i) the antibody or antigen-binding fragment thereof comprises: the following three heavy chain CDRs: a VH CDR1 with the sequence as set forth in SEQ ID NO: 5, a VH CDR2 with the sequence as set forth in SEQ ID NO: 6, and a VH CDR3 with the sequence as set forth in SEQ ID NO: 7; and/or, the following three light chain CDRs: a VL CDR1 with the sequence as set forth in SEQ ID NO: 8, a VL CDR2 with the sequence as set forth in SEQ ID NO: 9, a VL CDR3 with the sequence as set forth in SEQ ID NO: 10; and/or,
(ii) the antibody or antigen-binding fragment thereof comprises: three CDRs contained in the heavy chain variable region (VH) as set forth in SEQ ID NO: 3 or 60; and/or, three CDRs contained in the light chain variable region (VL) as set forth in SEQ ID NO: 4 or 61. In certain embodiments, the three CDRs contained in the VH and/or the three CDRs contained in the VL are defined by the Kabat, IMGT or Chothia numbering system.

In certain embodiments, the antibody or antigen-binding fragment thereof comprises:
a VH comprising the sequence as set forth in SEQ ID NO: 3 or variant thereof, and/or, a VL comprising the sequence as set forth in SEQ ID NO: 4 or variant thereof;
wherein, the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion, or addition of one or more amino acids (e.g., a substitution, deletion or addition of 1, 2, 3, 4, 5, 6, 7, 8, 9, or 10 amino acids) as compared to the sequence from which it is derived. In certain embodiments, the substitution is a conservative substitution.

In certain embodiments, the antibody or antigen-binding fragment thereof comprises: a VH as set forth in SEQ ID NO: 3, and/or a VL as set forth in SEQ ID NO: 4.

In certain embodiments, the antibody or antigen-binding fragment thereof is humanized, comprising a framework sequence derived from a human immunoglobulin. In certain embodiments, the antibody or antigen-binding fragment thereof comprises: a heavy chain framework sequence derived from a human heavy chain germline sequence, and a light chain framework sequence derived from a human light chain germline sequence, and the heavy chain framework sequence and/or the light chain framework sequence optionally comprise a back mutation from a human residue to a murine residue. In certain embodiments, the antibody or antigen-binding fragment thereof comprises: a VH as set forth in SEQ ID NO: 60, and/or a VL as set forth in SEQ ID NO: 61.

In a third aspect, the present application also provides an antibody or antigen-binding fragment thereof, which competes with the antibody or antigen-binding fragment thereof described in the second aspect (e.g., monoclonal antibody 5B11 or antigen-binding fragment thereof) to bind to RSV pre-F protein. In certain embodiments, the competitive binding refers to being able to block at least 50%, preferably at least 60%, preferably at least 70%, preferably at least 80%, preferably at least 90%, preferably at least 95% or preferably at least 99% the binding of the antibody or antigen-binding fragment thereof described in the second aspect (e.g., monoclonal antibody 5B11 or antigen-binding fragment thereof) to the RSV pre-F protein. The competitive binding can be determined by the competitive binding assays described above.

In certain embodiments, the epitope recognized by the antibody or antigen-binding fragment thereof is the same as, or overlaps in space with, the epitope recognized by the antibody or antigen-binding fragment thereof described in the second aspect (e.g., monoclonal antibody 5B11 or antigen-binding fragment thereof), such that the antibody or antigen-binding fragment thereof can block the binding of the antibody or antigen-binding fragment thereof described in the second aspect (e.g., monoclonal antibody 5B11 or antigen-binding fragment thereof) to the RSV pre-F protein. In certain embodiments, the antibody or antigen-binding fragment thereof binds to the same epitope of the RSV pre-F protein as the antibody or antigen-binding fragment thereof described in the second aspect (e.g., monoclonal antibody 5B11 or antigen-binding fragment thereof).

### 6B2

In a fourth aspect, the present application provides an antibody or antigen-binding fragment thereof that specifically binds to an epitope of respiratory syncytial virus (RSV) pre-F protein, wherein the epitope comprises at least 5 amino acid residues (e.g., non-consecutive amino acid residues) within the amino acid residues at positions 160-185 and 290-295 of the pre-F protein.

In certain embodiments, the epitope comprises at least the amino acid residues at positions 161, 162, 184, 293 and 294.

In certain embodiments, the epitope comprises the amino acid residues at positions 161-184 and the amino acid residues at positions 293-294.

In certain embodiments, the epitope is a conformational epitope.

In certain embodiments, the position of amino acid residue is determined according to SEQ ID NO: 1.

In certain embodiments, the epitope comprises at least E161, G162, G184, K293, and E294.

In a fifth aspect, the present application provides an antibody or antigen-binding fragment thereof, comprising:
(a) a heavy chain variable region (VH) comprising the following three CDRs: a VH CDR1 comprising the sequence as set forth in SEQ ID NO: 13 or variant thereof, a VH CDR2 comprising the sequence as set forth in SEQ ID NO: 14 or variant thereof, a VH CDR3 comprising the sequence as set forth in SEQ ID NO: 15 or variant thereof; and/or,
(b) a light chain variable region (VL) comprising the following three CDRs: a VL CDR1 comprising the sequence as set forth in SEQ ID NO: 16 or variant thereof, a VL CDR2 comprising the sequence as set forth in SEQ ID NO: 17 or variant thereof, a VL CDR3 comprising the sequence as set forth in SEQ ID NO: 18 or variant thereof;
wherein, the variant has a sequence identity of at least 70%, at least 80%, at least 85%, at least 90%, at least 91%, at least 92%, at least 93%, at least 94%, at least 95% %, at least 96%, at least 97%, at least 98%, at least 99%, or 100% as compared to the sequence from which it is derive, or the variant has a substitution, deletion, or addition of one or several amino acids (e.g., a substitution, deletion or addition of 1, 2 or 3 amino acids) as compared to the sequence from which it is derive. In certain embodiments, the substitution is a conservative substitution.

In certain embodiments, the antibody or antigen-binding fragment thereof is capable of specifically binding to RSV pre-F protein.

In certain embodiments, the antibody or antigen-binding fragment thereof competes with the antibody or antigen-binding fragment thereof of the fourth aspect to bind to the RSV pre-F protein. In certain embodiments, the competitive binding refers to being able to block at least 50%, preferably at least 60%, preferably at least 70%, preferably at least 80%, preferably at least 90%, preferably at least 95% or preferably at least 99% the binding of the antibody or antigen-binding fragment thereof described in the fourth aspect to the RSV pre-F protein. The competitive binding can be determined by the competitive binding assays described above.

In certain embodiments, the epitope recognized by the antibody or antigen-binding fragment thereof is the same as, or overlaps in space with, the epitope recognized by the antibody or antigen-binding fragment thereof described in the fourth aspect, such that the antibody or antigen-binding fragment thereof can block the binding of the antibody or antigen-binding fragment thereof of the fourth aspect to the RSV pre-F protein.

In certain embodiments, the antibody or antigen-binding fragment thereof binds to the same epitope of the RSV pre-F protein as the antibody or antigen-binding fragment thereof of the fourth aspect.

In certain embodiments:
(i) the antibody or antigen-binding fragment thereof comprises: the following three heavy chain CDRs: a VH CDR1 with the sequence as set forth in SEQ ID NO: 13, a VH CDR2 with the sequence as set forth in SEQ ID NO: 14, and a VH CDR3 with the sequence as set forth in SEQ ID NO: 15; and/or, the following three light chain CDRs: a VL CDR1 with the sequence as set forth in SEQ ID NO: 16, a VL CDR2 with the sequence as set forth in SEQ ID NO: 17, and a VL CDR3 with the sequence as set forth in SEQ ID NO: 18; and/or,
(ii) the antibody or antigen-binding fragment thereof comprises: three CDRs contained in the heavy chain variable region (VH) as set forth in SEQ ID NO: 11; and/or, three CDRs contained in the light chain variable region (VL) as set forth in SEQ ID NO: 12. In certain embodiments, the three CDRs contained in the VH and/or the three CDRs contained in the VL are defined by the Kabat, IMGT or Chothia numbering system.

In certain embodiments, the antibody or antigen-binding fragment thereof comprises:
a VH comprising the sequence as set forth in SEQ ID NO: 11 or variant thereof, and/or a VL comprising the sequence as set forth in SEQ ID NO: 12 or variant thereof;
wherein, the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion, or addition of one or more amino acids (e.g., a substitution, deletion or addition of 1, 2, 3, 4, 5, 6, 7, 8, 9, or 10 amino acids) as compared to the sequence from which it is derived. In certain embodiments, the substitution is a conservative substitution.

In certain embodiments, the antibody or antigen-binding fragment thereof comprises: a VH as set forth in SEQ ID NO: 11, and/or a VL as set forth in SEQ ID NO: 12.

In the sixth aspect, the present application also provides an antibody or antigen-binding fragment thereof, which competes with the antibody or antigen-binding fragment thereof described in the fifth aspect (e.g., monoclonal antibody 6B2 or antigen-binding fragment thereof) to bind to the RSV pre-F protein. In certain embodiments, the competitive binding refers to being able to block at least 50%, preferably at least 60%, preferably at least 70%, preferably at least 80%, preferably at least 90%, preferably at least 95% or preferably at least 99% the binding of the antibody or antigen-binding fragment thereof described in the fifth aspect (e.g., monoclonal antibody 6B2 or antigen-binding fragment thereof) to the RSV pre-F protein. The competitive binding can be determined by the competitive binding assays described above.

In certain embodiments, the epitope recognized by the antibody or antigen-binding fragment thereof is the same as, or overlaps in space with, the epitope recognized by the antibody or antigen-binding fragment thereof described in the fifth aspect (e.g., monoclonal antibody 6B2 or antigen-binding fragment thereof), such that the antibody or antigen-binding fragment thereof can block the binding of the antibody or antigen-binding fragment thereof described in the fifth aspect (e.g., monoclonal antibody 6B2 or antigen-binding fragment thereof) to the RSV pre-F protein. In certain embodiments, the antibody or antigen-binding fragment thereof binds to the same epitope of the RSV pre-F protein as the antibody or antigen-binding fragment thereof described in the fifth aspect (e.g., monoclonal antibody 6B2 or antigen-binding fragment thereof).

### 7G5

In a seventh aspect, the present application provides an antibody or antigen-binding fragment thereof that specifically binds to an epitope of respiratory syncytial virus (RSV) pre-F protein, wherein the epitope comprises at least 3 amino acid residues (e.g., non-consecutive amino acid residues) within the amino acid residues at positions 160-185 of the pre-F protein.

In certain embodiments, the epitope comprises at least the amino acid residues at positions 161, 162, and 184.

In certain embodiments, the epitope comprises the amino acid residues at positions 161-184.

In certain embodiments, the epitope is a conformational epitope.

In certain embodiments, the position of amino acid residue is determined according to SEQ ID NO: 1.

In certain embodiments, the epitope comprises at least E161, G162, and G184.

In an eighth aspect, the present application provides an antibody or antigen-binding fragment thereof, comprising:
(a) a heavy chain variable region (VH) comprising the following three CDRs: a VH CDR1 comprising the sequence as set forth in SEQ ID NO: 21 or variant thereof, a VH CDR2 comprising the sequence as set forth in SEQ ID NO: 22 or variant thereof, a VH CDR3 comprising the sequence as set forth in SEQ ID NO: 23 or variant thereof; and/or,
(b) a light chain variable region (VL) comprising the following three CDRs: a VL CDR1 comprising the sequence as set forth in SEQ ID NO: 24 or variant thereof, a VL CDR2 comprising the sequence as set forth in SEQ ID NO: 25 or variant thereof, a VL CDR3 comprising the sequence as set forth in SEQ ID NO: 26 or variant thereof;
wherein, the variant has a sequence identity of at least 70%, at least 80%, at least 85%, at least 90%, at least 91%, at least 92%, at least 93%, at least 94%, at least 95% %, at least 96%, at least 97%, at least 98%, at least 99%, or 100% as compared to the sequence from which it is derived, or the variant has a substitution, deletion, or addition of one or several amino acids (e.g., a substitution, deletion or addition of 1, 2 or 3 amino acids) as compared to the sequence from which it is derived. In certain embodiments, the substitution is a conservative substitution.

In certain embodiments, the antibody or antigen-binding fragment thereof is capable of specifically binding to the RSV pre-F protein.

In certain embodiments, the antibody or antigen-binding fragment thereof competes with the antibody or antigen-binding fragment thereof of the seventh aspect to bind to the RSV pre-F protein. In certain embodiments, the competitive binding refers to being able to block at least 50%, preferably at least 60%, preferably at least 70%, preferably at least 80%, preferably at least 90%, preferably at least 95% or preferably at least 99% the binding of the antibody or antigen-binding fragment thereof described in the seventh aspect to the RSV pre-F protein. The competitive binding can be determined by the competitive binding assays described above.

In certain embodiments, the epitope recognized by the antibody or antigen-binding fragment thereof is the same as, or overlaps in space with, the epitope recognized by the antibody or antigen-binding fragment thereof described in the seventh aspect, such that the antibody or antigen-binding fragment thereof can block the binding of the antibody or antigen-binding fragment thereof as described in the seventh aspect to the RSV pre-F protein.

In certain embodiments, the antibody or antigen-binding fragment thereof binds to the same epitope of the RSV pre-F protein as the antibody or antigen-binding fragment thereof of the seventh aspect.

In certain embodiments:
(i) the antibody or antigen-binding fragment thereof comprises: the following three heavy chain CDRs: a VH CDR1 with the sequence as set forth in SEQ ID NO: 21, a VH CDR2 with the sequence as set forth in SEQ ID NO: 22, and a VH CDR3 with the sequence as set forth in SEQ ID NO: 23; and/or, the following three light chain CDRs: a VL CDR1 with the sequence as set forth in SEQ ID NO: 24, a VL CDR2 with the sequence as set forth in SEQ ID NO: 25, and a VL CDR3 with the sequence as set forth in SEQ ID NO: 26; and/or,
(ii) the antibody or antigen-binding fragment thereof comprises: three CDRs contained in the heavy chain variable region (VH) as set forth in SEQ ID NO: 19; and/or, three CDRs contained in the light chain variable region (VL) as set forth in SEQ ID NO: 20. In certain embodiments, the three CDRs contained in the VH and/or the three CDRs contained in the VL are defined by the Kabat, IMGT or Chothia numbering system.

In certain embodiments, the antibody or antigen-binding fragment thereof comprises:
a VH comprising the sequence as set forth in SEQ ID NO: 19 or variant thereof, and/or a VL comprising the sequence as set forth in SEQ ID NO: 20 or variant thereof;
wherein the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletions or addition of one or several amino acids (e.g., a substitution, deletion or addition of 1, 2, 3, 4, 5, 6, 7, 8, 9, or 10 amino acids) as compared to the sequence from which it is derived. In certain embodiments, the substitution is a conservative substitution.

In certain embodiments, the antibody or antigen-binding fragment thereof comprises: a VH as set forth in SEQ ID NO: 19, and/or a VL as set forth in SEQ ID NO: 20.

In the ninth aspect, the present application also provides an antibody or antigen-binding fragment thereof, which competes with the antibody or antigen-binding fragment thereof described in the eighth aspect (e.g., monoclonal antibody 7G5 or antigen-binding fragment thereof) to bind to the RSV pre-F protein. In certain embodiments, the competitive binding refers to being able to block at least 50%, preferably at least 60%, preferably at least 70%, preferably at least 80%, preferably at least 90%, preferably at least 95% or preferably at least 99% the binding of the antibody or antigen-binding fragment thereof described in the fifth aspect (e.g., monoclonal antibody 7G5 or antigen-binding fragment thereof) to the RSV pre-F protein. The competitive binding can be determined by the competitive binding assays described above.

In certain embodiments, the epitope recognized by the antibody or antigen-binding fragment thereof is the same as, or overlaps in space with, the epitope recognized by the antibody or antigen-binding fragment thereof described in the eighth aspect (e.g., monoclonal antibody 7G5 or antigen-binding fragment thereof), such that the antibody or antigen-binding fragment thereof can block the binding of the antibody or antigen-binding fragment thereof described in the eighth aspect (e.g., monoclonal antibody 7G5 or antigen-binding fragment thereof) to the RSV pre-F protein. In certain embodiments, the antibody or antigen-binding fragment thereof binds to the same epitope of RSV pre-F protein as the antibody or antigen-binding fragment thereof described in the eighth aspect (e.g., monoclonal antibody 7G5 or antigen-binding fragment thereof).

In certain embodiments of any of the above aspects, the antibody or antigen-binding fragment thereof is derived from a mouse. In certain embodiments of any of the above aspects, the antibody or antigen-binding fragment thereof comprises a framework region sequence derived from a murine immunoglobulin.

In certain embodiments of any of the above aspects, the antibody or antigen-binding fragment thereof is humanized. In certain embodiments of any of the above aspects, the antibody or antigen-binding fragment thereof comprises a framework region sequence derived from a human immunoglobulin. In certain embodiments of any of the above aspects, the antibody or antigen-binding fragment thereof comprises: a heavy chain framework region sequence derived from a human heavy chain germline sequence, and a light chain framework region sequence derived from a human light chain germline sequence. The heavy chain framework region and/or light chain framework region optionally comprises a back mutation from a human residue to a murine residue.

In certain embodiments of any of the above aspects, the antibody or antigen-binding fragment thereof further comprises a constant region derived from a murine or human immunoglobulin.

In certain embodiments of any of the foregoing aspects, the heavy chain of the antibody or antigen-binding fragment thereof comprises a heavy chain constant region derived from a murine or human immunoglobulin (e.g., IgG1, IgG2, IgG3, or IgG4), and the light chain of the antibody or antigen-binding fragment thereof comprises a light chain constant region derived from a murine or human immunoglobulin (e.g., an immunoglobulin comprising λ or κ chain).

In certain embodiments, the heavy chain of the antibody or antigen-binding fragment thereof comprises a heavy chain constant region as set forth in SEQ ID NO: 62, and the light chain of the antibody or antigen-binding fragment thereof comprises a light chain constant region as set forth in SEQ ID NO: 63.

In certain embodiments of any of the above, the antigen-binding fragment is selected from the group consisting of Fab, Fab', (Fab')₂, Fv, disulfide-linked Fv, scFv, diabody, and single domain antibody (sdAb).

In certain embodiments of any of the above aspects, the antibody is a murine antibody, a chimeric antibody, a humanized antibody, a bispecific antibody, or a multispecific antibody.

In certain embodiments of any of the above aspects, the antibody or antigen-binding fragment thereof possesses one or more of the following characteristics:
(a) neutralizing an RSV (e.g., RSV type A and/or type B) in vitro or in a subject (e.g., a human);
(b) blocking or inhibiting the fusion of an RSV (e.g., RSV type A and/or type B) with a cell in vitro or in a subject (e.g., a human);
(c) preventing and/or treating an RSV (e.g., RSV type A and/or type B) infection or a disease associated with an RSV (e.g., RSV type A and/or type B) infection (e.g., pneumonia, such as pediatric pneumonia).

In certain embodiments, exemplary strains of RSV type A include virus strains as set forth in GenBank: KT992094.1, GU591760.1, KJ627328.1, KJ723478.1, KU316139.1, KU316112.1, FJ614813.1, KJ627352.1, KJ627274.1, KU316092.1, and/or HQ317235.1.

In certain embodiments, exemplary strains of RSV type B include virus strains as set forth in GenBank: JN032119.1, KX765905.1, KY249659.1, KJ627302.1, JQ736675.1, KX765900.1, JF714712.1, AF013254.1 and/or AY353550.1, and/or, the RSV 18537 strain.

### Isolated nucleic acid molecules

In a tenth aspect, the present application provides an isolated nucleic acid molecule encoding the antibody or antigen-binding fragment thereof as described in any of the above aspects, or heavy chain variable region and/or light chain variable region thereof.

In certain embodiments, the isolated nucleic acid molecule comprises a first nucleotide sequence encoding the heavy chain or heavy chain variable region of the antibody or antigen-binding fragment thereof of any aspect of the present application and a second nucleotide sequence encoding the light chain or light chain variable region of the antibody or antigen-binding fragment thereof, wherein the first nucleotide sequence and the second nucleotide sequence are present on the same or different isolated nucleic acid molecules. When the first nucleotide sequence and the second nucleotide sequence are present on different isolated nucleic acid molecules, the isolated nucleic acid molecule described herein comprises a first nucleic acid molecule comprising the first nucleotide sequence and a second nucleic acid molecule comprising the second nucleotide sequence.

### Vectors

In an eleventh aspect, the present application provides a vector, which comprises the nucleic acid molecule as described above. In certain embodiments, the vector is a cloning vector or an expression vector.

In certain embodiments, the vector comprises a first nucleotide sequence encoding the heavy chain or heavy chain variable region of the antibody or antigen-binding fragment thereof of any aspect of the present application and a second nucleotide sequence encoding the light chain or light chain variable region of the antibody or antigen-binding fragment thereof, wherein the first nucleotide sequence and the second nucleotide sequence are present on the same or different vectors. When the first nucleotide sequence and the second nucleotide sequence are present on different vectors, the vector described herein comprises a first vector comprising the first nucleotide sequence and a second vector comprising the second nucleotide sequence.

### Host cells

In a twelfth aspect, the present application provides a host cell, which comprises the nucleic acid molecule or the vector as described above. Such host cells include, but are not limited to, prokaryotic cells such as bacterial cells (e.g., *Escherichia coli* cells), and eukaryotic cells such as fungal cells (e.g., yeast cells), insect cells, plant cells, and animal cells (e.g., mammalian cells, such as mouse cells, human cells, etc.).

### Preparation methods

The antibodies of the present application can be prepared by various methods known in the art, such as by genetic engineering and recombinant technologies. For example, the DNA molecules encoding the heavy chain and light chain of the antibody of the present application are obtained through chemical synthesis or PCR amplification. The resulting DNA molecule is inserted into an expression vector and then transfected into host cells. Then, the transfected host cells are cultured under specific conditions and express the antibody of the present application.

The antigen-binding fragments of the present application can be obtained by hydrolyzing intact antibody molecules (see, Morimoto et al., J. Biochem. Biophys. Methods 24:107-117 (1992), and Brennan et al., Science 229:81 (1985)). Besides, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from the culture of recombinant host cells. Those of ordinary skill in the art are well aware of other techniques for preparing such antigen-binding fragments.

In a thirteenth aspect, the present application provides a method for preparing the antibody or antigen-binding fragment thereof of any aspect of the present application, which comprises culturing the host cell as described above under conditions that allow the expression of the antibody or antigen-binding fragment thereof, and recovering the antibody or antigen-binding fragment thereof from a culture of the cultured host cell.

### Pharmaceutical compositions

In a fourteenth aspect, the present application provides a pharmaceutical composition, which comprises the antibody or antigen-binding fragment thereof of any aspect of the present application, and a pharmaceutically acceptable carrier and/or excipient.

In certain exemplary embodiments, the pharmaceutically acceptable carrier and/or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution and any combination thereof.

### Uses for manufacturing medicaments

In the fifteenth aspect, the present application provides a use of the antibody or antigen-binding fragment thereof of any aspect of the present application in the manufacture of a medicament, in which the medicament is used for neutralizing the virulence of RSV, or inhibiting or blocking the fusion of RSV to a cell, or preventing and/or treating an RSV infection or a disease associated with an RSV infection (e.g., pneumonia, such as pediatric pneumonia) in a subject.

In certain embodiments, the subject is a mammal, such as a human.

In certain embodiments, the antibody or antigen-binding fragment thereof is used alone or in combination with an additional pharmaceutically active agent.

### Methods for prevention and/or treatment of diseases

In a sixteenth aspect, the present application provides a method for preventing and/or treating an RSV infection or a disease associated with an RSV infection (e.g., pneumonia, such as pediatric pneumonia) in a subject (e.g., a human), which comprises: administering to the subject in need thereof an effective amount of the antibody or antigen-binding fragment thereof of any aspect of the present application or the pharmaceutical composition of the present application.

The antibody or antigen-binding fragment thereof or pharmaceutical composition of the present application can be formulated into any dosage form known in the medical field, for example, tablets, pills, suspensions, emulsions, solutions, gels, capsules, powders, granules, elixirs, tablets, suppositories, injections (including injectable solutions, sterile powders for injection and concentrated solutions for injection), inhalants, sprays, etc. The preferred dosage form depends on the intended mode of administration and therapeutic use. The antibody or antigen-binding fragment thereof or pharmaceutical composition of the present application should be sterile and stable under the conditions of production and storage. One preferred dosage form is an injectable solution. Such injectable solution may be a sterile injectable solution. For example, the sterile injectable solution may be prepared by incorporating in an appropriate solvent the requisite dose of the antibody or antigen-binding fragment thereof of the present application, and, optionally, other desired ingredients including, but not limited to, pH adjusting agent, surfactant, adjuvant, ionic strength enhancing agent, isotonic agent, preservative, diluent, or any combination thereof, followed by filter sterilization. Additionally, the sterile injectable solution may be prepared as a sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution and any combination thereof.

The antibody or antigen-binding fragment thereof of the present application, or the pharmaceutical composition of the present application can be administered by any suitable method known in the art, including but not limited to, oral, buccal, sublingual, ophthalmic, local, parenteral, rectal, intrathecal, intracytoplasmic reticulum, inguinal, intravesical, topical (e.g., powder, ointment, or drops), or nasal route. However, for many therapeutic uses, the preferred route/mode of administration is parenteral administration (e.g., intravenous or bolus injection, subcutaneous injection, intraperitoneal injection, intramuscular injection). The skilled artisan will understand that the route and/or mode of administration will vary depending on the intended purpose. In certain embodiments, the antibody or antigen-binding fragment thereof or pharmaceutical composition of the present application is administered by intravenous injection or bolus injection.

### Conjugates

In a seventeenth aspect, the present application provides a conjugate, which comprises the antibody or antigen-binding fragment thereof of any aspect of the present application, and a detectable label linked to the antibody or antigen-binding fragment thereof.

In certain embodiments, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide and biotin.

### Kits

In the eighteenth aspect, the present application provides a kit, which comprises the antibody or antigen-binding fragment thereof of any aspect of the present application or the conjugate of the present application.

In certain embodiments, the kit comprises the conjugate of the present application.

In certain embodiments, the kit comprises the antibody or antigen-binding fragment thereof of any aspect of the present application, and optionally a second antibody that specifically recognizes the antibody or antigen-binding fragment thereof. Optionally, the second antibody further comprises a detectable label, such as an enzyme (e.g., horseradish peroxidase or alkaline phosphatase), a chemiluminescent reagent (e.g., acridinium ester, luminol and derivative thereof, or ruthenium derivative), fluorescent dye (e.g., fluorescein or fluoresceinprotein), radionuclide or biotin.

### Detection methods

In a nineteenth aspect, the present application provides a method for detecting the presence or level of RSV in a sample, which comprises using the antibody or antigen-binding fragment thereof of any aspect of the present application or the conjugate of the present application.

In certain embodiments, the method is used for therapeutic purposes, diagnostic purposes, or non-therapeutic non-diagnostic purposes.

In certain embodiments, the method is an immunological assay, such as a Western blotting assay, an enzyme immunoassay (e.g., ELISA), a chemiluminescent immunoassay, a fluorescent immunoassay, or a radioimmunoassay.

In certain embodiments, the method comprises using the conjugate of the present application.

In certain embodiments, the method comprises using the antibody or antigen-binding fragment thereof of any aspect of the present application, and the method further comprises using a second antibody carrying a detectable label (e.g., an enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide or biotin) to detect the antibody or antigen-binding fragment thereof.

In certain embodiments, the method comprises: (1) contacting the sample with the antibody or antigen-binding fragment thereof or the conjugate; (2) detecting the formation of an antigen-antibody immune complex or detecting an amount of the immune complex. In certain embodiments, the formation of immune complex indicates the presence of RSV or RSV-infected cells.

### Uses for manufacturing kits

In a twentieth aspect, the present application provides a use of the antibody or antigen-binding fragment thereof of any aspect of the present application or the conjugate of the present application in the manufacture of a kit, in which the kit is used for detecting the presence or level of RSV in a sample, and/or used for diagnosing whether a subject is infected with RSV.

In certain embodiments, the kit detects the presence or level of RSV in the sample by the method described in the nineteenth aspect.

In certain embodiments, the sample is a body fluid sample (e.g., a secretion of respiratory tract) or a tissue sample (e.g., a sample of respiratory tract tissue) from a subject (e.g., a mammal, preferably a human).

### Definition of Terms

In the present application, unless otherwise stated, scientific and technical terms used herein have the meanings commonly understood by those skilled in the art. Moreover, the virology, biochemistry, and immunology laboratory procedures used herein are routine procedures widely used in the corresponding fields. Meanwhile, in order to better understand the present application, definitions and explanations of relevant terms are provided below.

When the terms "for example," "such as," "e.g.," "comprising," "including," or variations thereof are used herein, these terms will not be considered limiting terms and will instead be interpreted to mean "but without limitation" or "without limitation."

Unless otherwise indicated herein or clearly contradicted by context, the terms "a" and "an" as well as "the" and similar referents in the context for describing the present application (especially in the context of the following claims) are to be construed to cover singular and plural.

As used herein, the term "RSV fusion protein" or "F protein" refers to a fusion protein (F protein) of respiratory syncytial virus (RSV), which is well known to those skilled in the art, and of which exemplary amino acid sequences can be found, for example, in UniProtKB: P03420.1. In the present application, when referring to the amino acid sequence of F protein, the sequence as set forth in SEQ ID NO: 1 is used for description. For example, the expression "amino acid residues at positions 160-182 of F protein" refers to amino acid residues at positions 160-182 of the polypeptide as set forth in SEQ ID NO: 1. However, those skilled in the art understand that in the amino acid sequence of the F protein, mutations or variations (including, but not limited to, substitution, deletion and/or addition, such as different genotypes or gene subtypes of the F protein) may be naturally occurring or artificially introduced without affecting its biological functions. Therefore, in the present application, the term "F protein" shall include all such sequences, including for example the sequence as set forth in SEQ ID NO: 1 and natural or artificial variants thereof. And, when describing the sequence fragment of F protein, it includes not only the sequence fragment of SEQ ID NO: 1, but also the corresponding sequence fragment in its natural or artificial variants. For example, the expression "amino acid residues at positions 160-182 of F protein" includes amino acid residues at positions 160-182 of SEQ ID NO: 1, and corresponding fragments in its (natural or artificial) variants. According to the present application, the expression "corresponding sequence fragment" or "corresponding fragment" refers to a fragment at equivalent positions located in the sequences when the sequences are compared for optimal alignment, that is, when the sequences are aligned to obtain the highest percent identity.

As used herein, the term "pre-F protein" refers to an F protein existing in a pre-F conformation. As used herein, the term "post-F protein" refers to an F protein existing in a post-F conformation. For more detailed descriptions of pre-F protein, post-F protein and their conformations, please refer to McLellan et al. (2010), J Vriol, 84: 12236-12244; McLellan et al. (2013), Science, 340: 1113-1117; McLellan et al. (2015), Curr Opin Virol, 11: 70-75; Chinese patent application 201480013927.7, and PCT international application PCT/CN2014/073505 (the entire contents of which are incorporated herein by reference for all purposes).

As used herein, the term "epitope" refers to a portion of an antigen capable of being recognized and specifically bound by a particular antibody. When the antigen is a polypeptide, the epitope can be formed from consecutive amino acids or non-consecutive amino acids neighbored by the tertiary folding of the protein, called linear epitope or conformational epitope, respectively. The epitope formed from consecutive amino acids is typically retained upon protein denaturation, whereas the epitope formed from tertiary folding is typically lost upon protein denaturation. The epitope usually comprises at least 3, 4, 5, 6, 7, 8, 9, 10, 11, 12, 13, 14 or 15 consecutive or non-consecutive amino acids in a unique spatial conformation.

As used herein, the term "identity" is used to refer to the match of sequences between two polypeptides or between two nucleic acids. To determine the percent identity of two amino acid sequences or two nucleic acid sequences, the sequences are aligned for optimal comparison purposes (e.g., gaps may be introduced in the first amino acid sequence or nucleic acid sequence for best alignment with the second amino acid or nucleic acid sequence). The amino acid residues or nucleotides at the corresponding amino acid positions or nucleotide positions are then compared. Molecules are identical at a position when the position in the first sequence is occupied by the same amino acid residue or nucleotide as the corresponding position in the second sequence. The percent identity between two sequences is a function of the number of identical positions shared by the sequences (i.e., percent identity = number of identical overlapping positions/total number of positions × 100%). In certain embodiments, both sequences are of the same length.

Determination of percent identity between two sequences can also be accomplished using mathematical algorithms. One non-limiting example of mathematical algorithm for comparison of two sequences is the algorithm of Karlin and Altschul, 1990, Proc. Natl. Acad. Sci. U.S.A. 87:2264-2268, which was improved by Karlin and Altschul, 1993, Proc. Natl. Acad. Sci. U.S.A. 90:5873-5877. Such algorithms were integrated into the NBLAST and XBLAST programs of Altschul et al., 1990, J. Mol. Biol. 215:403.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and the antibody's isotypes are defined as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin with host tissue or factor, including various cells of the immune system (e.g., effector cells) and the first component of the classical complement system (C1q). The VH and VL regions can also be subdivided into regions of high variability called complementarity-determining regions (CDRs), interspersed with more conservative regions called framework regions (FRs). Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form an antigen-binding site. The assignment of amino acids to regions or domains can follow the definition as described in Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity-determining region" or "CDR" refers to those amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present application, the CDRs contained in the antibody or antigen-binding fragment thereof of the present application can be determined according to various numbering systems known in the art. In certain embodiments, the CDRs contained in the antibody or antigen-binding fragment thereof of the present application are preferably determined by the Kabat, Chothia, or IMGT numbering systems. In certain embodiments, the CDRs contained in the antibody or antigen-binding fragment thereof of the present application are preferably determined by the IMGT numbering system.

As used herein, the term "framework region" or "FR" residues refers to those amino acid residues in an antibody variable region other than the CDR residues as defined above.

The term "antibody" is not limited to any particular method of producing the antibody. It includes, for example, recombinant antibody, monoclonal antibody, and polyclonal antibody. The antibody may be of different isotypes, for example, IgG (e.g., IgG1, IgG2, IgG3 or IgG4 subtypes), IgA1, IgA2, IgD, IgE or IgM antibody.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of an antibody can be obtained by recombinant DNA technology or by enzymatic or chemical cleavage of an intact antibody. Non-limiting examples of antigen-binding fragments include Fab, Fab', F(ab')₂, Fd, Fv, complementarity-determining region (CDR) fragment, scFv, diabody, single domain antibody, chimeric antibody, linear antibody, nanobody (technology from Domantis), probody, and such polypeptides, which contain at least a portion of an antibody that is sufficient to confer specificity to the polypeptides with antigen-binding capability. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains." Among them, "full-length heavy chain" refers to a polypeptide chain that consists of a heavy chain variable region (VH), a heavy chain constant region CH1 domain, a hinge region (HR), a heavy chain constant region CH2 domain, a heavy chain constant region CH3 domain in the direction from the N-terminal to the C-terminal; and, when the full-length antibody is of IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and between the HRs of the two full-length heavy chains. The full-length antibody of the present application can be from a single species, such as human; it can also be a chimeric antibody or a humanized antibody. The full-length antibody of the present application contains two antigen-binding sites formed by VH and VL pairs respectively, and these two antigen-binding sites specifically recognize/bind the same antigen.

As used herein, the term "Fd" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')₂ fragment" refers to an antibody fragment consisting of two Fab fragments connected by disulfide bridges on the hinge region; the term "Fab' fragment" refers to a fragment obtained by reducing the disulfide bond connecting the two heavy chain fragments in the F(ab')₂ fragment, and consists of a complete light chain and heavy chain Fd fragment (consisting of VH and CH1 domains).

As used herein, the term "Fv" refers to an antibody fragment consisting of VL and VH domains of a single arm of an antibody. Fv fragments are generally considered to be the smallest antibody fragments that can form a complete antigen-binding site. It is generally believed that six CDRs confer the antigen-binding specificity of an antibody. However, even a variable region (e.g., an Fd fragment, which contains only three antigen-specific CDRs) can recognize and bind the antigen, although its affinity may be lower than that of the intact binding site.

As used herein, the term "Fc" refers to an antibody fragment formed by bonding of the second and third constant regions of a first heavy chain of an antibody to the second and third constant regions of a second heavy chain of an antibody via disulfide bonds. The Fc fragment of an antibody has many different functions but does not participate in antigen binding.

As used herein, the term "scFv" refers to a single polypeptide chain comprising VL and VH domains, in which the VL and VH are connected via a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH₂-VL-linker-VH-COOH or NH₂-VH-linker-VL-COOH. Suitable prior art linkers consist of repeated GGGGS amino acid sequences or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers useful in the present application are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol. In some cases, a disulfide bond may also exist between VH and VL of scFv. In certain embodiments of the present application, scFv can form di-scFv, which refers to an antibody formed by connecting two or more individual scFvs in series. In certain embodiments of the present application, scFv can form (scFv)₂, which refers to an antibody formed by connecting two or more individual scFvs in parallel.

As used herein, the term "diabody" refers to an antibody whose VH and VL domains are expressed on a single polypeptide chain, but the linker used is too short to allow pairing between the two domains on the same chain, which forces the domains to pair with the complementary domains of the other chains and generate two antigen-binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the common meaning as generally understood by those skilled in the art, and refers to an antibody fragment consisting of a single monomeric variable antibody domain (e.g., a single heavy chain variable region), which retains the ability to specifically bind to the same antigen to which the full-length antibody binds. The single domain antibody is also called nanobody.

Each of the above antibody fragments retains the ability to specifically bind to the same antigen to which the full-length antibody binds, and/or competes with the full-length antibody for specific binding to the antigen.

Antigen-binding fragments of antibody (e.g., the above-described antibody fragments) can be obtained from a given antibody (e.g., the antibody provided by the present application) using conventional techniques known to those skilled in the art (e.g., recombinant DNA technology or enzymatic or chemical fragmentation methods), and the antigen-binding fragments of the antibody are screened for specificity in the same manner as for intact antibodies.

As used herein, when the term "antibody" is mentioned, it includes not only an intact antibody but also an antigen-binding fragment of the antibody, unless the context clearly indicates otherwise.

As used herein, the term "chimeric antibody" refers to an antibody in which a part of the light chain and/or heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), nevertheless, it still retains the binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include such an antibody in which the heavy and light chain variable regions of the antibody are derived from a first antibody and the heavy and light chain constant regions of the antibody are derived from a second antibody.

As used herein, the term "variant", in the context of polypeptides (including polypeptides), also refers to a polypeptide or peptide comprising an amino acid sequence that has been altered by introducing a substitution, deletion, or addition of amino acid residues. In some cases, the term "variant" also refers to a polypeptide or peptide that has been modified (i.e., by covalently linking any type of molecule to the polypeptide or peptide). For example, and without limitation, polypeptides may be modified, for example, by glycosylation, acetylation, PEGylation, phosphorylation, amidation, derivatization by known protecting/blocking groups, proteolytic cleavage, attachment to cellular ligand or other proteins, etc. Derivatized polypeptides or peptides can be produced by chemical modification using techniques known to those skilled in the art, including, but not limited to, specific chemical cleavage, acetylation, formylation, metabolic synthesis of tunicamycin, and the like. Furthermore, a variant has a similar, identical or improved function to the polypeptide or peptide from which it is derived.

As used herein, the term "specific binding" refers to a non-random binding reaction between two molecules, such as the reaction between an antibody and an antigen to which is directed. The strength or affinity of a specific binding interaction can be expressed by the equilibrium dissociation constant (K_{D}) of the interaction. In the present application, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding properties between two molecules can be determined using methods known in the art. One approach involves measuring the rate at which antigen binding site/antigen complex forms and dissociates. Both the "association rate constant" (kₐ or kₒₙ) and the "dissociation rate constant" (k_{dis} or k_{off}) can be calculated from concentrations and actual rates of association and dissociation (see Malmqvist M, Nature, 1993, 361 : 186-187). The ratio k_{dis}/kₒₙ is equal to the dissociation constant K_{D} (see Davies et al., Annual Rev Biochem, 1990; 59:439-473). K_{D}, kₒₙ and k_{dis} values can be measured by any valid method. In certain embodiments, dissociation constants can be measured by surface plasmon resonance (SPR) with a Biacore instrument. Besides, bioluminescence interferometry or Kinexa can be used to measure dissociation constants.

As used herein, a detectable label of the present application may be any substance detectable by fluorescent, spectroscopic, photochemical, biochemical, immunological, electrical, optical or chemical means. Such labels are well known in the art and examples include, but are not limited to, enzymes (e.g., horseradish peroxidase, alkaline phosphatase, beta-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g., Cy7, Alexa 750)), luminescent substances (e.g., chemiluminescent substances, such as acridinium esters, luminol and its derivatives compounds, ruthenium derivatives such as ruthenium terpyridyl), magnetic beads (e.g., Dynabeads^{®}), calorimetric markers such as colloidal gold or colored glass or plastic (e.g., polystyrene, polypropylene, latex, etc.) beads, and biotin for binding to avidin (e.g., streptavidin) modified with the above label.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); phages, such as λ phage or M13 phage and animal viruses, etc. Animal viruses that can be used as vectors include, but are not limited to, retroviruses (including lentiviruses), adenoviruses, adeno-associated viruses, herpesviruses (e.g., herpes simplex virus), poxviruses, baculoviruses, papillomaviruses, papovaviruses (e.g., SV40). A vector can contain a variety of expression-controlling elements, including, but not limited to, promoter sequence, transcription initiation sequence, enhancer sequence, selection element, and reporter gene. In addition, the vector may also contain an origin of replication.

As used herein, the term "host cell" refers to a cell that can be used to introduce a vector, which includes, but is not limited to, prokaryotic cell such as *E. coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to substitute the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which is incorporated herein by reference).

The twenty conventional amino acids involved herein have been written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present application, the terms "polypeptide" and "protein" have the same meaning and are used interchangeably. And in the present application, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, being not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes sterile injectable liquid (e.g., aqueous or non-aqueous suspensions or solutions). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

As used herein, the term "prevention" refers to a method performed to prevent or delay the occurrence of a disease or condition or symptom in a subject. As used herein, the term "treatment" refers to a method performed to obtain a beneficial or desired clinical outcome. For the purposes of this application, beneficial or desired clinical outcome includes, but is not limited to, alleviation of symptoms, reduction of the extent of disease, stabilization (i.e., no worsening) of the state of disease, delaying or slowing the progression of disease, ameliorating or alleviating the status of disease, and relief of symptoms (whether partial or complete), whether detectable or undetectable. In addition, "treatment" may also refer to prolonging survival compared to expected survival if not receiving the treatment.

As used herein, the term "subject" refers to a mammal, such as a human. In certain embodiments, the subject (e.g., a human) has, or is at risk for, an RSV infection or a disease associated with an RSV infection (e.g., pneumonia, such as pediatric pneumonia).

As used herein, the term "effective amount" refers to an amount sufficient to achieve, or at least partially achieve, the desired effect. For example, an effective amount to prevent a disease (e.g., an RSV infection or a disease associated with an RSV infection (e.g., pneumonia, such as pediatric pneumonia)) refers to an amount sufficient to prevent, arrest, or delay the occurrence of the disease; an effective amount to treat a disease refers to an amount sufficient to cure or at least partially prevent a disease and its complications in a patient who is already suffering from the disease. Determining such effective amounts is well within the capabilities of those skilled in the art. For example, the amount effective for therapeutic use will depend on the severity of the disease to be treated, the overall status of the patient's own immune system, the patient's general condition such as age, body weight and gender, the mode for drug administration, and other treatments administered concurrently, and so on.

### Beneficial effects of the present application

The monoclonal antibody and antigen-binding fragment thereof of the present application have broad-spectrum RSV-binding activity and can specifically bind to the F proteins of multiple RSV strains of type-A and/or type-B, and the monoclonal antibodies and antigen-binding fragments thereof can specifically bind to the new epitopes located between the antigenic site Ø and site V or the new epitopes located between the antigenic site II and site V on the pre-F proteins, and thus have high RSV neutralizing activity and can effectively prevent an RSV infection in vivo.

The monoclonal antibodies and antigen-binding fragments thereof of the present application can be used to detect, prevent and/or treat an RSV infection or a disease caused by an RSV infection. In addition, the antibodies of the present application can also be used to specifically detect an RSV or F protein thereof, and has clinical values in the diagnosis of an RSV infection.

The embodiments of the present application will be described in detail below with reference to the accompanying drawings and examples, but those skilled in the art will understand that the following drawings and examples are only used to illustrate the present application and do not limit the scope of the present application. Various objects and advantageous aspects of the present application will become apparent to those skilled in the art from the accompanying drawings and the following detailed description of preferred embodiments.

### Brief Description of the Drawings

Fig. 1 shows the experimental results of monoclonal antibodies 5B11, 6B2 and 7G5 neutralizing the representative strain of RSV type A (RSV A2) (panel A) and the representative strain of RSV type B (18537) (panel B).
Fig. 2 shows the ELISA reactivity of monoclonal antibodies 5B11, 6B2 and 7G5 with the prefusion and postfusion proteins of the representative strain of RSV type A and the representative strain of RSV type B; wherein panels A and B show the ELISA reactivity results of each antibody with the prefusion and postfusion proteins of the representative strain of RSV type A, respectively; panels C and D show the ELISA reactivity results of each antibody with the prefusion and postfusion proteins of the representative strain of RSV type B, respectively.
Fig. 3 shows the broad-spectrum binding of monoclonal antibodies 5B11, 6B2 and 7G5 to the F proteins of different type A and type B RSV strains.
Fig. 4 shows the three-dimensional structures of monoclonal antibodies 5B11, 6B2 and 7G5 bound to RSV F protein in the prefusion conformation (DS-Cav1).
Fig. 5 shows the key amino acid residues involved in the binding of monoclonal antibodies 5B11, 6B2 and 7G5 in the RSV F protein of prefusion conformation (DS-Cav1).
Fig. 6 shows the cryo-electron microscopy three-dimensional reconstruction results of the binding of monoclonal antibody 5B11 to RSV F protein in prefusion conformation (DS-Cav1).
Fig. 7 shows the protective effect of monoclonal antibodies 5B11, 6B2 and 7G5 in preventing RSV A2 infection in vivo in mice. Wherein, panel A shows the body weight monitoring results of mice in each group; panel B shows the results of plaques in the lung tissue of mice in each group; panel C shows the results of plaques in the nasal tissue of mice in each group; panel D shows the staining results of pathological sections of the lung tissue of mice in each group.

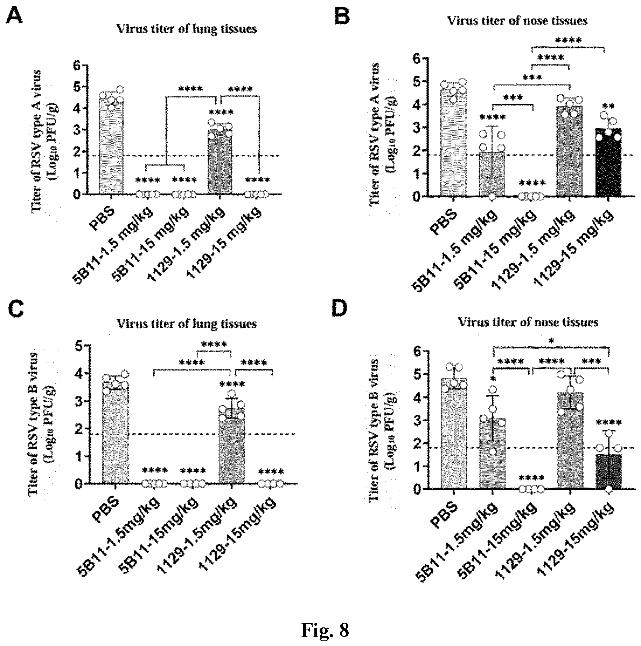

Fig. 8 shows the nose and lung virus titers in the evaluation of preventive effects of monoclonal antibody 5B11 on RSV type A and type B viruses (the strains used for type A and type B were RSV A2 and 18537, respectively) in cotton rat model. Wherein, panel A shows the lung virus titer detection results of cotton rats in each group in the evaluation experiment of using 5B11 to prevent RSV type A virus; panel B shows the nose virus titer detection results of cotton rats in each group in the evaluation experimental of using 5B11 to prevent RSV type A virus; panel C shows the lung virus titer detection results of cotton rats in each group in the evaluation experiment of using 5B11 to prevent RSV type B virus; panel D shows the nose virus titer detection results of cotton rats in each group in the evaluation experiment of using 5B11 to prevent RSV type B virus.
Fig. 9 shows the pathological scores of lung tissues in the evaluation of preventive effects of 5B11 on RSV type A and type B viruses (the strains used for type A and type B were RSV A2 and 18537, respectively) in cotton rat model. Wherein, panels A to E show the pathological scores of lung tissues of cotton rats in each group in the evaluation experiment of using 5B11 to prevent RSV type A virus; panels F to J show the pathological scores of lung tissues of cotton rats in of each group in the evaluation experiment of using 5B11 to prevent RSV type B virus.
Fig. 10 shows the design scheme of humanization of 5B11.
Fig. 11 shows the detection for neutralization and reactivity of humanized 5B11 antibody; wherein panel A shows the detection results of humanized antibody N5B11 in neutralizing RSV type A virus (RSV A2); panel B shows the detection results of humanized antibody N5B11 in neutralizing RSV type B virus (18537); panel C shows the binding detection results of humanized antibody N5B11 to RSV type A (RSV A2) pre-F; and panel D shows the binding detection results of humanized antibody N5B11 to RSV type B (18537) pre-F.

### Sequence information

The table below provides a description of the sequences involved in the present application.

**Table 1: Sequence information**

| SEQ ID NO: | Description of sequence |
|---|---|
| 1 | Amino acid sequence of RSV A2 F protein |
| | MELLILKANAITTILTAVTFCFASGQNITEEFYQSTCSAVSKGYLSALRTGW YTSVITIELSNIKKNKCNGTDAKIKLIKQELDKYKNAVTELQLLMQSTPAT NNRARRELPRFMNYTLNNAKKTNVTLSKKRKRRFLGFLLGVGSAIASGVA VSKVLHLEGEVNKIKSALLSTNKAVVSLSNGVSVLTSKVLDLKNYIDKQL LPIVNKQSCSISNIETVIEFQQKNNRLLEITREFSVNAGVTTPVSTYMLTNSE LLSLINDMPITNDQKKLMSNNVQIVRQQSYSIMSIIKEEVLAYVVQLPLYG VIDTPCWKLHTSPLCTTNTKEGSNICLTRTDRGWYCDNAGSVSFFPQAETC KVQSNRVFCDTMNSLTLPSEVNLCNVDIFNPKYDCKIMTSKTDVSSSVITS LGAIVSCYGKTKCTASNKNRGIIKTFSNGCDYVSNKGVDTVSVGNTLYYV NKQEGKSLYVKGEPIINFYDPLVFPSDEFDASISQVNEKINQSLAFIRKSDEL LHNVNAVKSTTNIMITTIIIVIIVILLSLIAVGLLLYCKARSTPVTLSKDQLSG INNIAFSN |
| 2 | Nucleotide sequence encoding RSV A2 F protein |
| | ATGGAGTTGCTAATCCTCAAAGCAAATGCAATTACCACAATCCTCACT GCAGTCACATTTTGTTTTGCTTCTGGTCAAAACATCACTGAAGAATTTT ATCAATCAACATGCAGTGCAGTTAGCAAAGGCTATCTTAGTGCTCTGA GAACTGGTTGGTATACCAGTGTTATAACTATAGAATTAAGTAATATCA AGAAAAATAAGTGTAATGGAACAGATGCTAAGGTAAAATTGATAAAA CAAGAATTAGATAAATATAAAAATGCTGTAACAGAATTGCAGTTGCTC ATGCAAAGCACACAAGCAACAAACAATCGAGCCAGAAGAGAACTACC AAGGTTTATGAATTATACACTCAACAATGCCAAAAAAACCAATGTAAC ATTAAGCAAGAAAAGGAAAAGAAGATTTCTTGGTTTTTTGTTAGGTGT TGGATCTGCAATCGCCAGTGGCGTTGCTGTATCTAAGGTCCTGCACCTA GAAGGGGAAGTGAACAAGATCAAAAGTGCTCTACTATCCACAAACAA GGCTGTAGTCAGCTTATCAAATGGAGTTAGTGTTTTAACCAGCAAAGT GTTAGACCTCAAAAACTATATAGATAAACAATTGTTACCTATTGTGAA CAAGCAAAGCTGCAGCATATCAAATATAGAAACTGTGATAGAGTTCCA ACAAAAGAACAACAGACTACTAGAGATTACCAGGGAATTTAGTGTTAA TGCAGGCGTAACTACACCTGTAAGCACTTACATGTTAACTAATAGTGA ATTATTGTCATTAATCAATGATATGCCTATAACAAATGATCAGAAAAA GTTAATGTCCAACAATGTTCAAATAGTTAGACAGCAAAGTTACTCTATC ATGTCCATAATAAAAGAGGAAGTCTTAGCATATGTAGTACAATTACCA CTATATGGTGTTATAGATACACCCTGTTGGAAACTACACACATCCCCTC TATGTACAACCAACACAAAAGAAGGGTCCAACATCTGTTTAACAAGAA CTGACAGAGGATGGTACTGTGACAATGCAGGATCAGTATCTTTCTTCCC ACAAGCTGAAACATGTAAAGTTCAATCAAATCGAGTATTTTGTGACAC AATGAACAGTTTAACATTACCAAGTGAAGTAAATCTCTGCAATGTTGA CATATTCAACCCCAAATATGATTGTAAAATTATGACTTCAAAAACAGA TGTAAGCAGCTCCGTTATCACATCTCTAGGAGCCATTGTGTCATGCTAT GGCAAAACTAAATGTACAGCATCCAATAAAAATCGTGGAATCATAAAG ACATTTTCTAACGGGTGCGATTATGTATCAAATAAAGGGGTGGACACT GTGTCTGTAGGTAACACATTATATTATGTAAATAAGCAAGAAGGTAAA AGTCTCTATGTAAAAGGTGAACCAATAATAAATTTCTATGACCCATTA GTATTCCCCTCTGATGAATTTGATGCATCAATATCTCAAGTCAACGAGA AGATTAACCAGAGCCTAGCATTTATTCGTAAATCCGATGAATTATTACA TAATGTAAATGCTGGTAAATCCACCACAAATATCATGATAACTACTAT AATTATAGTGATTATAGTAATATTGTTATCATTAATTGCTGTTGGACTG CTCTTATACTGTAAGGCCAGAAGCACACCAGTCACACTAAGCAAAGAT CAACTGAGTGGTATAAATAATATTGCATTTAGTAACTAA |
| 3 | Amino acid sequence of 5B11 VH |
| | QIQLVQSGPELKKPGETVKISCKASGYTFTDYSMHWLKQAPGKGLKWMG WITTETGEPTYADDFKGRFAFSLETSASTAYLQINNLKNEDTGIYFCARYY YGPFYWGQGTLVTVST |
| 4 | Amino acid sequence of 5B11 VL |
| | DIQMTQSPASLSASVGETVTITCRSSGNIHNFLTWYQQKQGKSPQFLVYNA KTLADGVSSRFSGSGSGTQFSLKINSLQPEDFGIYYCQHFWTTPYTFGGGT KLEIK |
| 5 | Amino acid sequence of 5B11 VH CDR1 |
| | GYTFTDYS |
| 6 | Amino acid sequence of 5B11 VH CDR2 |
| | ITTETGEP |
| 7 | Amino acid sequence of 5B11 VH CDR3 |
| | ARYYYGPFY |
| 8 | Amino acid sequence of 5B11 VL CDR1 |
| | GNIHNF |
| 9 | Amino acid sequence of 5B11 VL CDR2 |
| | NAK |
| 10 | Amino acid sequence of 5B11 VL CDR3 |
| | QHFWTTPYT |
| 11 | Amino acid sequence of 6B2 VH |
| | EVQLQQSRPELVKPGASVKISCKASGYSFTAYFMNWVKQSHGKSLEWIGR INPYIGDTFYNQKFKGKATLTVDKSSNTAHMELLSLTSEDSAVYYCGRSE YGNYYFDYWGQGTTLTVSQ |
| 12 | Amino acid sequence of 6B2 VL |
| | ENVLTQSPAIMSASLGEKVTMSCRASSSVNYMYWYQQKSDASPKLWIYY TSNLAPGVPARFSGSGSGNSYSLTISSMEGEDAATYYCQQFTSSPLTFGAG TKLELK |
| 13 | Amino acid sequence of 6B2 VH CDR1 |
| | GYSFTAYF |
| 14 | Amino acid sequence of 6B2 VH CDR2 |
| | INPYIGDT |
| 15 | Amino acid sequence of 6B2 VH CDR3 |
| | GRSEYGNYYFDY |
| 16 | Amino acid sequence of 6B2 VL CDR1 |
| | SSVNY |
| 17 | Amino acid sequence of 6B2 VL CDR2 |
| | YTS |
| 18 | Amino acid sequence of 6B2 VL CDR3 |
| | QQFTSSPLT |
| 19 | Amino acid sequence of 7G5 VH |
| | QVQLKESGPGLVAPSQSLSITCTVSGFSLSSYGVHWIRQPPGKGLEWLGVI WAGGSTNYNSALMSRLSISEDNSKSQVFLKMNSLQTDDTAVYYCARKGL VWPAMDYWGQGTSVTVSQ |
| 20 | Amino acid sequence of 7G5 VL |
| | DIQMTQTTSSLSASLGDRVTISCRASQDISHYLNWYQQKPDGTVKLLIYYT SRLHSGVPSRFSGSGSGTDYSLTIYNLEQEDIATYFCQQGNTLPWTFGGGT KLEIK |
| 21 | Amino acid sequence of 7G5 VH CDR1 |
| | GFSLSSYG |
| 22 | Amino acid sequence of 7G5 VH CDR2 |
| | IWAGGST |
| 23 | Amino acid sequence of 7G5 VH CDR3 |
| | ARKGLVWPAMDY |
| 24 | Amino acid sequence of 7G5 VL CDR1 |
| | QDISHY |
| 25 | Amino acid sequence of 7G5 VL CDR2 |
| | YTS |
| 26 | Amino acid sequence of 7G5 VL CDR3 |
| | QQGNTLPWT |
| 27 | Nucleotide sequence of primer MVJkR |
| | CCGTTTGKATYTCCAGCTTGGTSCC |
| 28 | Nucleotide sequence of primer MVDJhR |
| | CGGTGACCGWGGTBCCTTGRCCCCA |
| 29-59 | Amplification primer of variable region |
| 60 | Amino acid sequence of N5B11 VH |
| | QIQLVQSGPELKKPGASVKISCKASGYTFTDYSMHWLKQAPGKGLKWMG WITTETGEPTYADDFKGRFAFSLDTSASTAYLQISSLKAEDTGVYFCARYY YGPFYWGQGTLVTVSS |
| 61 | Amino acid sequence of N5B11 VL |
| | DIQMTQSPSSLSASVGDRVTITCRSSGNIHNFLTWYQQKPGKSPQFLVYNA KTLADGVPSRFSGSGSGTQFTLTISSLQPEDFGIYYCQHFWTTPYTFGGGT KVEIK |
| 62 | Amino acid sequence of human immunoglobulin heavy chain constant region |
| | |
| 63 | Amino acid sequence of human immunoglobulin light chain constant region |
| | |

| | |
|---|---|
| Note: K=G, or T; Y=C or T; S=C or G; W=A or T; B=C, G or T; R=A or G. | |

### Specific Models for Carrying Out the present application

The present application will now be described with reference to the following examples which are intended to illustrate but not to limit the present application.

Unless otherwise specified, the molecular biology experimental methods and immunoassay methods used in the present application are carried out basically according to the method described by J. Sambrook et al., Molecular Cloning: Laboratory Manual, 2nd Edition, Cold Spring Harbor Laboratory Press, 1989, and F. M. Ausubel et al., Compiled Laboratory Guide to Molecular Biology, 3rd Edition, John Wiley & Sons, Inc., 1995; the uses of restriction enzymes are carried out according to the conditions recommended by the product manufacturers. If the specific conditions were not specified in the examples, the conditions should be carried out according to the conventional conditions or the conditions recommended by the manufacturers. If the manufacturers of the reagents or instruments used were not indicated, they were all conventional products that could be purchased commercially. Those skilled in the art will appreciate that the examples describe the present application by way of example and are not intended to limit the scope of the present application.

### Example 1: Preparation of monoclonal antibody (McAb) against RSV-F protein

(1) Preparation of immunogen:
   The plasmid DNA-F was kindly donated by the NIH Vaccine Research Center in the United States. In this plasmid, the full-length gene of F protein (GenBank: FJ614814.1, SEQ ID NO: 1) of the RSV A2 strain was inserted into the VRC8400 vector. In the present application, an endotoxin-free plasmid maximal extraction kit (purchased from TianGen, Cat. No.: DP117) was used to obtain a low endotoxin DNA-F at a concentration of 2 mg/mL as an immunogen for mouse immunization. At the same time, in the present application, a recombinant adenovirus (rAd-F) containing the full-length gene of F protein (SEQ ID NO: 1) was also prepared for mouse immunization. This recombinant adenovirus was a virus that had been constructed and cryopreserved in our laboratory. For the needs of mouse immunization, the cryopreserved virus was quickly thawed and used to infect 293β5 cells (239 cells stably transduced with the human β5 gene, which were constructed and stored by our laboratory). The virus was harvested 48 hours after the infection, and concentrated to 1 × 10¹⁰ PFU/mL by density gradient centrifugation for the later mouse immunization.
(2) Experimental mice:
   Six-week-old SPF grade female Balb/C mice were purchased from Shanghai Slaccas Laboratory Animal Co., Ltd.
(3) Preparation of hybridoma:
   Standard in vivo immunization method and PEG fusion method were used to obtain monoclonal antibody hybridoma cells, in which the detailed methods were referred to Ed Harlow et al., "Antibodies A Laboratory Manual", Cold Spring Harbor Laboratory 1988. The brief process was described as follows:
(4) Mouse immunization:
   The immunogen DNA-F (100 µg/mouse) prepared in the above step (1) or rAd-F (5×10⁷ PFU/mouse) were injected into the mice by high-pressure tail vein injection. The immunization cycle for DNA-F was 4 weeks, and the immunization cycle for rAd-F was 2 weeks. In order to boost immune response, the mice were injected subcutaneously with complete Freund's adjuvant during the primary immunization with DNA-F immunogen; and the booster immunization with rAd-F was performed by subcutaneous injection with incomplete Freund's adjuvant. Subsequently, blood was collected from mouse orbits every week, and then the reactivity and serum neutralization titer of the sera of mice in each group were detected, and the mice with higher serum neutralizing antibody titers were selected for spleen immunization. HEp-2 cells infected with RSV A2 for 48 hours were scraped off with a cell scraper, centrifuged to remove the MEM medium, and the cell pellet was resuspended in 500 µL of 1 ×PBS, and 50 µL of the suspension was taken for spleen immunization. After 3 days, the spleen was taken and ground, and spleen cells were isolated. The spleen cells were fused with mouse myeloma cells (SP2/0) using PEG.
(5) Cell fusion:
   First, the mouse spleen was taken and ground to obtain a suspension of spleen cells, and then mixed with the mouse myeloma cells SP2/0 in the logarithmic growth phase. Cell fusion was performed under the presence of PEG1500. The fused cells were resuspended in 400 mL of fusion medium, divided into aliquots, loaded into 20 96-well cell culture plates and cultured. The fusion medium was RPMI1640 complete selection medium containing HAT and 20% FBS.
(6) Screening of hybridoma:
   After the fused cells were cultured on the 96-well cell culture plates for 10 days, the cell supernatant was pipetted for virus neutralization experiments and ELISA detection, and the virus used for the detection was RSV A2 mKate. During the virus neutralization, the well in which the antibodies secreted could inhibit the infection of Hep2 cells by respiratory syncytial virus was defined as a positive well; during the ELISA detection, the well in which the antibodies secreted could specifically react with the respiratory syncytial virus-infected Hep2 cells fixed on the cell plates was defined as a positive well. After cloning the positive clones three times, a monoclonal antibody cell line capable of stably secreting antibodies was obtained.
(7) Results of screening hybridoma monoclonal antibody:
   Nine strains of anti-RSV neutralizing monoclonal antibodies specific for prefusion protein, including 5B11, 6B2 and 7G5, were obtained.
(8) Cultivation of hybridoma:
   The 9 stable hybridoma monoclonal antibody cell lines were expanded and cultured in a carbon dioxide incubator, then transferred from 96 wells to 24 wells, and then transferred into 50 mL cell culture bottles for expansion and culture. Then the cells in the cell bottles were collected and injected into the abdominal cavity of mice. After 7 to 10 days, the monoclonal antibody ascites was drawn from the abdominal cavity of mice.
(9) Purification of monoclonal antibodies:
   The monoclonal antibody ascites was precipitated with 50% ammonium sulfate solution, and then the precipitate was dissolved with PBS and purified through Protein A column under AKTA system to obtain the purified monoclonal antibody, which was identified by SDS-PAGE to determine the purity of the purified monoclonal antibody.

### Example 2: Neutralizing activity of monoclonal antibodies against RSV F protein

Neutralizing activity is an important indicator to evaluate whether a monoclonal antibody has the potential to prevent and treat diseases. The microwell cell neutralization experiment was used to detect the neutralizing activity of the monoclonal antibodies 5B11, 6B2 and 7G5 obtained in Example 1 against the representative strains of respiratory syncytial virus type A and type B, RSV A2 and 18537 (the method was referred to Yong-Peng Sun et al., Journal of Virological Methods. 2018, 260:34-40). The control antibodies used were 5C4 (a mouse-derived antibody screened by our laboratory, PCT No: PCT/CN2014/073505), D25 (expressed by the constructed plasmid, Patent No: US 8,568,726 B2) and 1129 (its sequence was donated by NIH, and it was expressed by the constructed plasmid).

**Table 2: Summary of IC50 of each RSV neutralizing antibody**

| mAbs | RSV A2 (ng/mL) | 18537 (ng/mL) |
|---|---|---|
| 4E10 | 785.5 | 101.9 |
| 5B11 | 7.4 | 59.3 |
| 6B2 | 94.9 | 97.2 |
| 6H7 | 175.2 | 241.3 |
| 11A12 | 101.8 | 178.4 |
| 11C1 | 525.1 | 233.9 |
| 12F8 | 132.2 | 91.9 |
| 7G5 | 31.6 | 103.8 |
| 10F3 | 43.1 | 125.7 |
| 5C4 | 9.4 | - |
| D25 | 22.2 | 167.2 |
| 1129 | 472.0 | 520.2 |

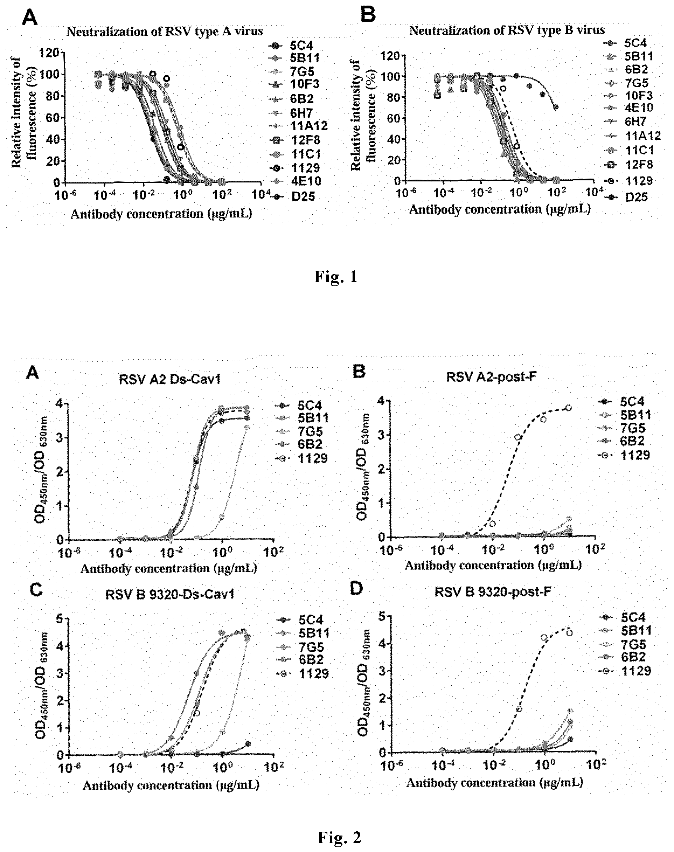

The results were shown in Table 2 and Fig. 1. The results showed that the monoclonal antibodies 5B11, 6B2 and 7G5 all showed better ability to neutralize RSV A2 and 18537 than 1129, in which the titer of 5B11 in neutralizing RSV A2 was the similar to that of the control antibody 5C4, and was more than 50 times the neutralizing ability of 1129, and the ability of 5B11 in neutralizing 18537 was about 10 times that of 1129.

### Example 3: ELISA detection of reactivity of anti-RSV F protein monoclonal antibody

For the procedures and methods of indirect ELISA detection of antigen-antibody binding, please refer to the prior art documents (Min Zhao et al., J Biol Chem. 2015, 290(32):19910-22). The detection results were shown in Fig. 2, in which the three monoclonal antibodies (5B11, 6B2 and 7G5) only bound to the F protein in pre-F conformation (DS-Cav1) of RSV type A and type B viruses and did not bind to the F protein in post-F conformation, and thus they are antibodies that specifically recognize epitopes specifically present on RSV pre-F.

### Example 4: Sequence analysis of light chain gene and heavy chain gene of anti-RSV F protein monoclonal antibody

About 10⁷ hybridoma cells underwent semi-adherent culture. The adherent cells were blown up and suspended, and the suspension was transferred to a new 4 mL centrifuge tube and centrifuged at 1500 rpm for 3 min. The cell pellet was collected and resuspended in 100 µL of sterile PBS (pH=7.45), and transferred to a new 1.5 mL centrifuge tube. 800 µL of Trizol (purchased from Roche, Germany) was added, mixed gently by turning upside down, and allowed to stand for 10 min. 200 µL of chloroform was added, shaken vigorously for 15 s, allowed to stand for 10 min, and centrifuged at 12000 rpm for 15 min at 4°C. The upper liquid was transferred to a new 1.5 mL centrifuge tube, added with an equal volume of isopropanol, mixed well, allowed to stand for 10 min, and centrifuged at 12000 rpm at 4°C for 10 min. The supernatant was discarded, 600 µL of 75% ethanol was added for washing, centrifugation was performed at 12000 rpm at 4°C for 5 min, and the supernatant was discarded. The precipitate was vacuum dried at 60°C for 5 min. The clear pellet was dissolved in 70 µL of DEPC H₂O and divided into two tubes. 1 µL of reverse transcription primer was added to each tube. The reverse transcription primer added to one tube was MVJkR (SEQ ID NO: 27), which was used to amplify the light chain variable region gene. The reverse transcription primer added to the other tube was MVDJhR (SEQ ID NO: 28), which was used to amplify the heavy chain variable region gene. 1 µL of dNTP (purchased from Shanghai Sangon) was added to each tube, and the tube was placed in a 72°C water bath for 10 minutes, then immediately placed in an ice bath for 5 minutes. 10 µL of 5x reverse transcription buffer, 1 µL of AMV (10U/µL, purchased from Pormega), 1µL of Rnasin (40U/µL, purchased from Promega) were added, mixed well to reverse-transcribe RNA into cDNA at 42°C.

The variable region of the antibody gene was isolated using the polymerase chain reaction (PCR) method, in which a primer set synthesized based on Novagen's Ig-Prime kits and two additionally designed and synthesized downstream primers MVJkR and MVDJhR (synthesized by Shanghai Sangon) were used, MVJkR was the downstream primer for light chain variable region gene amplification, and MVDJhR was the downstream primer for heavy chain variable region gene amplification. The templates were the two cDNAs synthesized above. PCR conditions were: 94°C 5min, 94°C 40s, 53°C 1min, 72°C 50s 35 cycles, and 72°C 15min. The PCR product was directly sent to Shanghai Sangon for sequencing. The sequence was compared with IMGT to determine the antibody variable region sequence and the corresponding amino acid sequence.

The antibody variable region genes were obtained by cloning from 5B11, 6B2 and 7G5 monoclonal antibody hybridoma cell lines according to the above method, and the amino acid sequences of CDR regions (complementary determinant regions) of the monoclonal antibodies were determined by referring to the IMGT method (Marie-PaµLe Lefranc and Gérard Lefranc. ImmunoglobµLins or Antibodies: IMGT® Bridging Genes, Structures and Functions, Biomedicines 2020, 8, 319). The variable regions and CDR sequences of each antibody (defined by the IMGT numbering system) were shown in Table 1. Table 3 showed the primer sequences as used.

**Table 3: Primer sequences used to amplify the variable region genes of 5B11, 6B2 and 7G5 monoclonal antibodies**

| Primer | SEQ ID NO: | Name | Sequence |
|---|---|---|---|
| Heavy chain PCR primer | 29 | MuIgVH5'-A | GGGAATTCATGRASTTSKGGYTMARCTKGRTTT |
| | 30 | MuIgVH5'-B | GGGAATTCATGRAATGSASCTGGGTYWTYCTCTT |
| | 31 | MuIgVHS'-C1 | ACTAGTCGACATGGACTCCAGGCTCAATTTAGTTTTCCT |
| | 32 | MuIgVH5'-C2 | ACTAGTCGACATGGCTGTCYTRGBGCTGYTCYTCTG |
| | 33 | MuIgVH5'-C3 | ACTAGTCGACATGGVTTGGSTGTGGAMCTTGCYATTCCT |
| | 34 | MuIgVH5'-D1 | ACTAGTCGACATGAAATGCAGCTGGRTYATSTTCTT |
| | 35 | MuIgVH5'-D2 | ACTAGTCGACATGGRCAGRCTTACWTYYTCATTCCT |
| | 36 | MuIgVH5'-D3 | ACTAGTCGACATGATGGTGTTAAGTCTTCTGTACCT |
| | 37 | MuIgVH5'-E1 | ACTAGTCGACATGGGATGGAGCTRTATCATSYTCTT |
| | 38 | MuIgVH5'-E2 | ACTAGTCGACATGAAGWTGTGGBTRAACTGGRT |
| | 39 | MuIgVH5'-E3 | ACTAGTCGACATGGRATGGASCKKIRTCTTTMTCT |
| | 40 | MuIgVH5'-F1 | ACTAGTCGACATGAACTTYGGGYTSAGMTTGRTTT |
| | 41 | MuIgVH5'-F2 | ACTAGTCGACATGTACTTGGGACTGAGCTGTGTAT |
| | 42 | MuIgVH5'-F3 | ACTAGTCGACATGAGAGTGCTGATTCTTTTGTG |
| | 43 | MuIgVH5'-F4 | ACTAGTCGACATGGATTTTGGGCTGATTTTTTTTATTG |
| Light chain PCR primer | 44 | MuIgκVL5'-A | GGGAATTCATGRAGWCACAKWCYCAGGTCTTT |
| | 45 | MuIgκVL5'-B | GGGAATTCATGGAGACAGACACACTCCTGCTAT |
| | 46 | MuIgκVL5'-C | ACTAGTCGACATGGAGWCAGACACACTSCTGYTATGGG T |
| | 47 | MuIgκVL5'-D1 | ACTAGTCGACATGAGGRCCCCTGCTCAGWTTYTTGGIW TCTT |
| | 48 | MuIgκVL5'-D2 | ACTAGTCGACATGGGCWTCAAGATGRAGTCACAKWYY CWGG |
| | 49 | MuIgκVL5'-E1 | ACTAGTCGACATGAGTGTGCYCACTCAGGTCCTGGSGT T |
| | 50 | MuIgκVL5'-E2 | ACTAGTCGACATGTGGGGAYCGKTTTYAMMCTTTTCAA TTG |
| | 51 | MuIgκVL5'-E3 | ACTAGTCGACATGGAAGCCCCAGCTCAGCTTCTCTTCC |
| | 52 | MuIgκVL5'-F1 | ACTAGTCGACATGAGIMMKTCIMTTCAITTCYTGGG |
| | 53 | MuIgκVL5'-F2 | ACTAGTCGACATGAKGTHCYCIGCTCAGYTYCTIRG |
| | 54 | MuIgκVL5'-F3 | ACTAGTCGACATGGTRTCCWCASCTCAGTTCCTTG |
| | 55 | MuIgκVL5'-F4 | ACTAGTCGACATGTATATATGTTTGTTGTCTATTTCT |
| | 56 | MuIgκVL5'-G1 | ACTAGTCGACATGAAGTTGCCTGTTAGGCTGTTGGTGCT |
| | 57 | MuIgκVL5'-G2 | ACTAGTCGACATGGATTTWCARGTGCAGATTWTCAGCT T |
| | 58 | MuIgκVL5'-G3 | ACTAGTCGACATGGTYCTYATVTCCTTGCTGTTCTGG |
| | 59 | MuIgκVL5'-G4 | ACTAGTCGACATGGTYCTYATVTTRCTGCTGCTATGG |

| | | | |
|---|---|---|---|
| Note: K=G or T; Y=C or T; S=C or G; W=A or T; B=C, G or T; R=A or G; M=A or C; H= A, C or T; V= A, C or G; I=hypoxanthine deoxyribonucleotide residue | | | |

### Example 5: Detection of broad-spectrum binding activity of anti-RSV F protein monoclonal antibodies

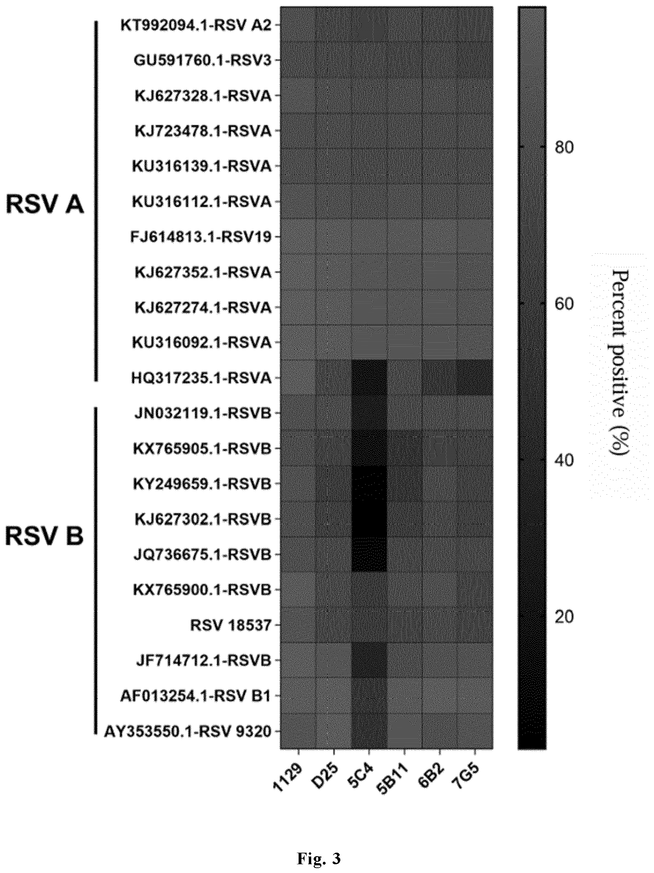

More than 1,000 nucleic acid sequences of RSV F protein were downloaded from NCBI, evolutionary tree analysis of RSV F protein was performed through MEGA software, 20 representative RSV F protein sequences were selected, and these 20 sequences were constructed into VRC8400 vector (synthesized by Shanghai Sangon). These plasmids were then transfected into 293 cells, these proteins were overexpressed on the cell membrane, and the binding of the 3 antibodies, 5B11, 6B2 and 7G5, to 293 cells overexpressing F protein was detected by flow cytometry. The experimental results were shown in Fig. 3. The results showed that 5B11, 6B2 and 7G5 all had strong broad-spectrum binding activities and could bind well to the F proteins of RSV type A and type B strains, so they were potential RSV broad-spectrum neutralizing antibodies.

### Example 6: Identification of three-dimensional structure of the complex of anti-RSV F protein monoclonal antibody and RSV prefusion protein (DS-Cav1), as well as the identification of key amino acid residues

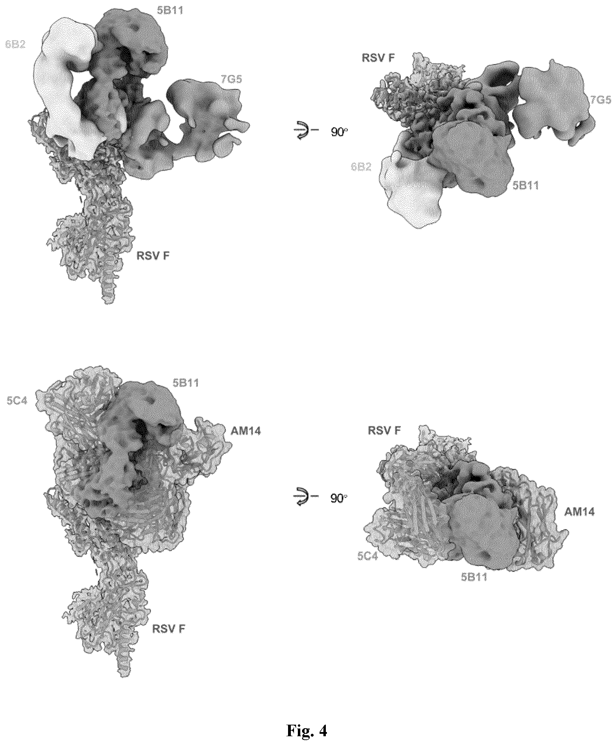

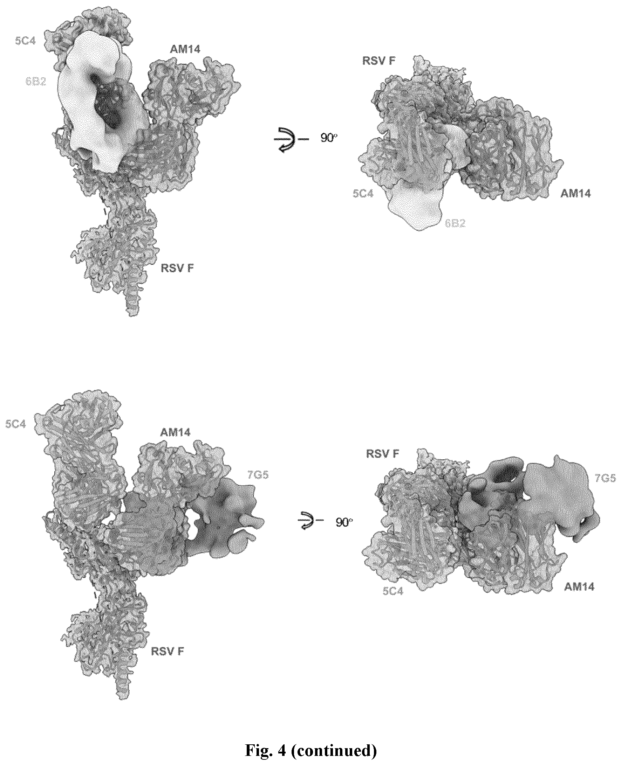

In order to clarify the binding epitope of three antibodies 5B11, 6B2 and 7G5 to the RSV prefusion protein, we prepared the antigen-antibody complexes of the RSV prefusion protein (DS-Cav1) with antibodies 5B11, 6B2 and 7G5 respectively, and analyzed the structures of these antigen-antibody complexes by three-dimensional reconstruction technology using cryo-electron microscopy. The experimental results were shown in Fig. 4. The results showed that the three antibodies 5B11, 6B2 and 7G5 bound to new epitopes on the RSV prefusion protein (DS-Cav1), in which the antibodies 5B11 and 6B2 could specifically bind to a new epitope located between the antigenic site Ø and site V on the respiratory syncytial virus pre-F protein, while antibody 7G5 could specifically bind to a new epitope located between the antigenic site II and site V on the respiratory syncytial virus pre-F protein.

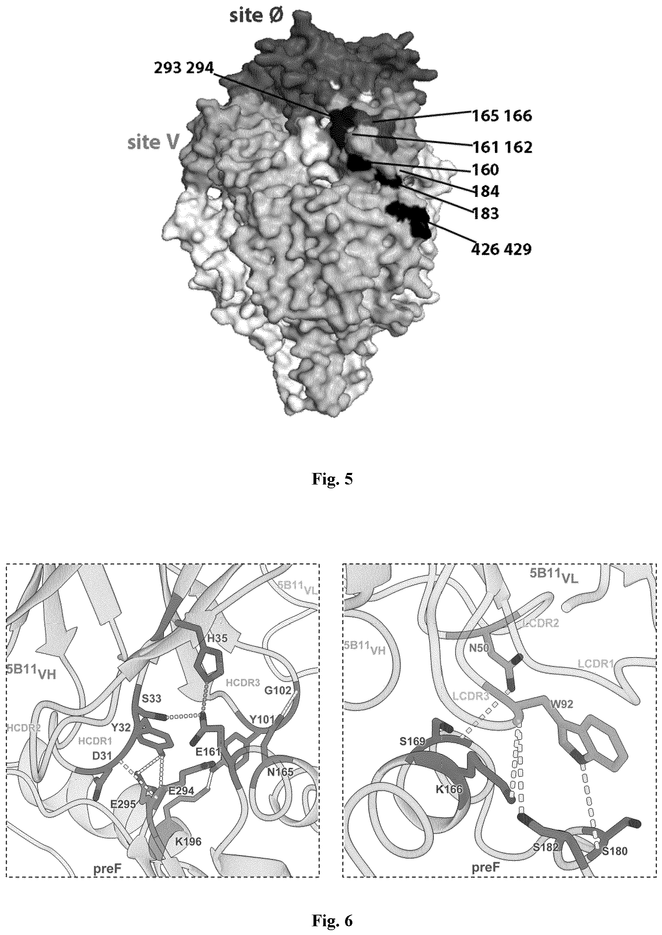

In order to further clarify the key amino acid residues involved in the binding of the three antibodies, alanine scanning was performed on the regions of F protein bound to the three antibodies, that was, the surface amino acids of the F protein were mutated into alanine (A), a mutant plasmid library was constructed by mutation based on DNA-F plasmids, the mutant plasmids were transfected into 293 cells, the F protein was overexpressed on the cell surface, and the binding of the three antibodies 5B11, 6B2 and 7G5 to these mutant proteins was detected by flow cytometry. The experimental results were shown in Fig. 5, which indicated that the key amino acid residues involved in the binding of SB11 were E161, G162, N165, K166 and G184, the key amino acid residues involved in the binding of 6B2 were E161, G162, G184, K293 and E294, and the key amino acid residues involved in the binding of 7G5 were E161, G162 and G184.

In addition, in order to further understand the structural basis of the interaction between antibody 5B11 and RSV F protein, we prepared 5B11 Fab-DS-Cav1 antigen-antibody complex and obtained a 3.29 angstrom-high resolution 5B11 Fab-DS-Cav1 antigen-antibody complex structure through cryo-electron microscopy three-dimensional reconstruction technology (Cryo-EM). The results were shown in Fig. 6. It could be seen from the structure that 5B11 bound to the F1 subunit on the F protein, and the main binding regions were aa. 160-182 and the loop between β6 and β7 (aa.294-295). The heavy chain of 5B11 mainly interacted with the RSV F protein through CDR1 and CDR3; specifically, D31 on CDR1 of the heavy chain of 5B11 formed a hydrogen bond with E295 on the F1 subunit, Y32 formed hydrogen bonds with E294 and E295, S33 formed a hydrogen bond with E161, and additionally, H35 on FR2 also interacted with E161 to form a hydrogen bond. Y101 on the heavy chain CDR3 of 5B11 formed a hydrogen bond with K196 on the F1 subunit, and G102 formed a hydrogen bond with N165. The light chain of SB11 mainly interacted with RSV F protein through CDR2 and CDR3; specifically, N50 on the heavy chain CDR2 of 5B11 interacted with S169 on the F1 subunit to form a hydrogen bond, and W92 on the heavy chain CDR3 of 5B11 interacted with K166, S180 and S182 on the F1 subunit to form hydrogen bonds.

### Example 7: Evaluation of preventive effects of anti-RSV F protein monoclonal antibody on RSV virus in Balb/C model

Passive antibody therapy is a potentially effective antiviral treatment approach for the treatment of infectious diseases. Through in vitro micropore neutralization experiments, it had been confirmed that the monoclonal antibodies 5B11, 6B2 and 7G5 of the present application had strong neutralizing activity against RSV type A and type B strains, and were characterized by broad neutralizing reaction spectrum and high neutralizing titer against respiratory syncytial virus type A and type B strains. In order to further verify the anti-respiratory syncytial virus effect of monoclonal antibodies 5B11, 6B2 and 7G5 in vivo, in the present application, based on the animal model of respiratory syncytial virus A2 strain infection, the in vivo validation experiments of the monoclonal antibodies in the prevention of RSV A2 strain were performed on Balb/C mice in a biosafety laboratory, and the details were as follows:
(1) Materials and methods
Animals: Balb/C mice, SPF grade, 12 weeks old, female, body weight about 20g.
Monoclonal antibodies: 5B11, 6B2, 7G5 and 1129
Respiratory syncytial virus A2 strains as purchased from ATCC.
Anesthetic: Isoflorane (isofurane)
Animal grouping: The mice were sent to the biosafety laboratory one week in advance to adapt to the environment. There were a total of 50 mice, 10 in each group, and 5 in each cage. The mice were marked and the body weight of each mouse was recorded. The detailed scheme was shown in Table 4.
Viral infection: The respiratory syncytial virus A2 strain was diluted to 5×10⁷ PFU/mL in advance, and the virus inoculation volume was 100 µL/mouse. Before inoculation, the mice were anesthetized with isoflurane, and then the virus was inoculated into the nasal cavity to infect the mice.
Monoclonal antibody intervention: The mice in the antibody prevention group were given a low-dose of antibody 24 hours before the viral infection for prevention. Each mouse was injected intraperitoneally with a dose of 1.5 mg/kg of antibody in a volume of 100 µL.
Observation and recording: From the 1^{st} to 12^{th} days after the viral infection, the changes in body weight and corresponding behavioral characteristics of the mice were recorded every day. On the 5^{th} day after infection, 5 mice from each group were selected to detect the virus titers in nose and lung tissues and the pathological characteristics of lung tissues.

**Table 4: Experimental scheme for evaluation of preventive effects of anti-RSV F protein antibodies in animals**

| Group | Remarks | Infection virus | Monoclonal antibody intervention | Number of mice |
|---|---|---|---|---|
| A | Positive control group | RSV A2 | PBS | 10 |
| B | RSV type A prevention group | RSV A2 | 5B11 1.5mg/mL | 10 |
| C | RSV type A prevention group | RSV A2 | 6B2 1.5mg/mL | 10 |
| D | RSV type A prevention group | RSV A2 | 7G5 1.5mg/mL | 10 |
| E | 1129 control group | RSV A2 | 1129 1.5mg/mL | 10 |

(2) Results and analysis

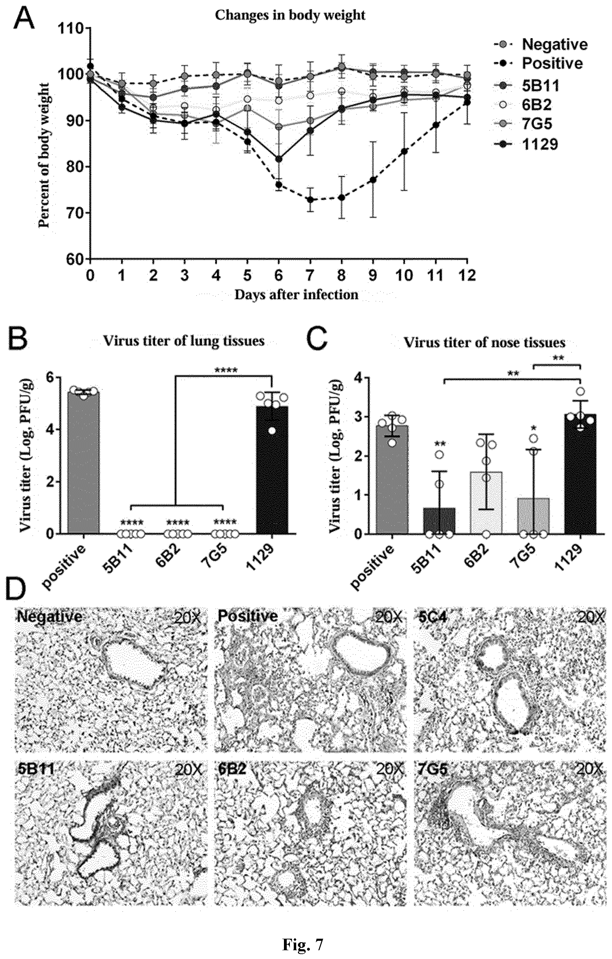

After being infected with RSV A2, a total of 50 mice in 5 experimental groups including the positive control group, 5B11, 6B2, 7G5 and 1129 groups were monitored every day for body weight and whether there was inverse hair phenomenon. The body weight monitoring results were shown in panel A of Fig. 7. The body weight of mice in the positive control group began to decrease on the 4^{th} day after infection, dropped to a low point on the 7^{th} day, and then gradually recovered. Compared with the 1129 control group, the 5B11, 6B2 and 7G5 groups did not show obvious weight loss, indicating that they could better protect the mice from weight loss to a certain extent, and their protective effects were better than that of the 1129 control group. During the experiment, it was found that the mice in the positive control group and the 1129 control group were found to have obvious reverse hair phenomenon on the 5^{th} day after infection, while no obvious reverse hair phenomenon was found in the other groups.

The results of virus plaques in nose and lung tissues were shown in panels C and B of Fig. 7 respectively. From the plaque detection results in lung tissues, it was found that the virus titers in the lung tissues of mice in the positive group and the 1129 control group were relatively high, reaching 10^{5.4} PFU/g and 10^{4.9} PFU/g, respectively; while no virus was detected in the 5B11, 6B2 and 7G5 groups, and there were significant statistical differences from the positive group and the 1129 control group (P < 0.05). In addition, the three strains of antibodies (5B11, 6B2 and 7G5) could also reduce viral infection in the upper respiratory tract and nose to a certain extent, and their effects were better than that of the 1129 control group (panel C in Fig. 7). In summary, the three strains of antibodies (5B11, 6B2 and 7G5) could prevent upper and lower respiratory tract infections at low doses.

Through the pathological characteristics of the lung tissue (panel D in Fig. 7), it could be seen that in the positive group and the 1129 control group, a large number of inflammatory cells infiltrated appeared on the walls of blood vessels, bronchioles, capillary bronchioles as well as alveolar walls of the mouse lung tissues, the swelling was significant, and in some severe cases, the cell cavities were compressed and the alveolar walls were broken. It could be seen from the pathological sections of the 6B2 and 7G5 antibody groups that the lung tissues contained slight pathological indications, the blood vessel walls, bronchiolar walls and capillary bronchiolar walls were thickened, slight inflammatory cell infiltration could be seen around them, but the alveolar structures were clear and complete, and there were no obvious inflammatory cell infiltration and thickening. In the mice of the 5B11 group, there was no inflammatory cell infiltration in the walls of blood vessels, bronchioles, capillary bronchioles as well as alveolar walls of the lung tissues, and their morphologies and structures were normal.

The above experimental results showed that the low-dose prevention experiments showed that at the same dose, the preventive effects of the three antibodies 5B11, 6B2 and 7G5 were better than that of the 1129 control group, indicating that they could effectively prevent RSV infection, reduce the weight loss and symptoms of mice, inhibit the virus replication in upper and lower respiratory tracts, and reduce inflammatory response, etc.

### Example 8: Evaluation of preventive effects of 5B11 monoclonal antibody on RSV virus in cotton rat model

### (1) Evaluation of preventive effects of 5B11 on RSV type A virus in cotton rat model

In order to explore whether 5B11 had the potential to become a preventive drug for RSV, in the present application, the evaluation of preventive effects of 5B11 on RSV type A virus in a cotton rat model was further performed. The grouping designed for the experiment was shown in Table 5, and there were 5 groups in total, 5 cotton rats in each group. For the antibody groups, the antibody was diluted in advance with PBS to 15 mg/mL and 1.5 mg/mL for later use according to high dose (15 mg/kg) and low dose (1.5 mg/kg); on the day before challenge (Day -1), each rat was intramuscularly injected with the antibody in a certain volume according to the rat body weight into the right hind limb; 24 hours later (Day 0), 100 µL of RSV A2 virus with a titer of 2×10⁷ PFU/mL was administered into the nasal cavity. For the PBS group, 100 µL of PBS was intramuscularly injected into the right hind limb of each cotton rat on the day before the challenge; 24 hours later, 100 µL of RSV A2 virus with a titer of 2×10⁷ PFU/mL was administered into the nasal cavity. On the 5^{th} day after the challenge, the cotton rats in each group were euthanized with CO₂, and nasal and lung tissues were collected by dissection for evaluation of virus titers in nose and lung tissues and lung pathological inflammation.

**Table 5: Grouping for experiments of evaluation of preventive effects of RSV F protein antibodies on cotton rats**

| Group | Infection virus | Drug intervention | Number of cotton rats |
|---|---|---|---|
| PBS group | RSV A2 | PBS | 5 |
| 5B11 high dose group | RSV A2 | 5B11 (15 mg/kg) | 5 |
| 5B11 low dose group | RSV A2 | 5B11 (1.5 mg/kg) | 5 |
| 1129 high dose group | RSV A2 | 1129 (15 mg/kg) | 5 |
| 1129 low dose group | RSV A2 | 1129 (1.5 mg/kg) | 5 |

The lung plaque detection results (panel A in Fig. 8) showed that no virus titer was detected in the lungs of the high and low dose groups of 5B11, suggesting that 5B11 could completely prevent RSV lung infection at low doses; for 1129, only the high dose group showed that no virus titer was detected, and RSV lung infection was completely prevented; while a higher virus titer was detected in the low-dose group of 1129, the viral infection was reduced by 25-fold (1.4 Log) as compared with the PBS group, indicating that it could only prevent the virus lung infection to a certain extent.

The nose virus plaque detection results (panel B in Fig. 8) showed that the high and low dose groups of 5B11 and the high dose group of 1129 all had significant statistical differences as compared with the PBS group. Wherein, the nasal RSV infection could be completely prevented in the high dose group of 5B11, and the viral infection was reduced by 500-fold (2.7 log) as compared with the PBS group. The viral infection was reduced by 5-fold (0.7 log) in the low dose group of 1129 as compared with the PBS group, and the viral infection was reduced by 50-fold (1.7 log) in the high dose group of 1129 as compared with the PBS group, indicating that none of them could completely prevent the nasal virus infection in the rats.

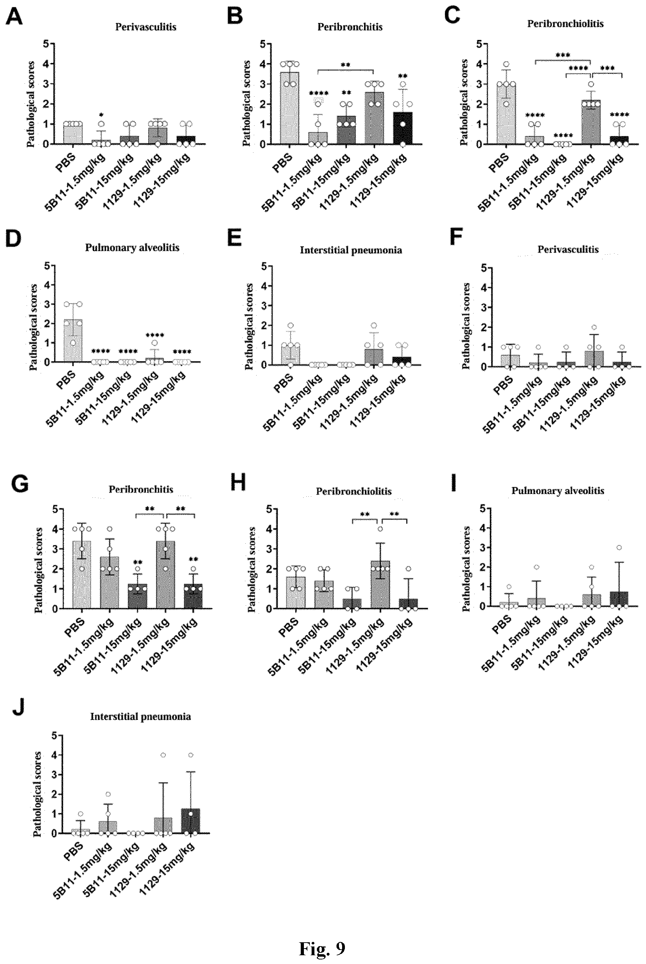

The pathological inflammation of lung tissues in each group was quantified and scored according to the degree of inflammatory cell infiltration at different parts, and the differences in inflammation scores between groups were calculated by the analysis of variance. The pathological scores of the lungs (panels A to E in Fig. 9) showed that the perivasculitis and interstitial inflammation in the lungs of cotton rats in each group were not obvious, while the lung inflammation in the PBS group mainly manifested as severe tracheitis, bronchitis, and pulmonary alveolitis. No obvious vasculitis, tracheitis, bronchitis, pulmonary alveolitis and interstitial pneumonia were found in the high and low dose groups of 5B11. The high and low dose groups of 1129 showed tracheitis and bronchitis, in which the tracheitis and bronchitis in the high dose group of 1129 were mild, while the tracheitis and bronchitis in the low dose group of 1129 were more serious. The above results suggested that 5B11 had good potential to inhibit lung inflammation, and a low dose of 5B11 could achieve or even surpass the ability of a high dose of 1129 to inhibit lung inflammation.

### (2) Evaluation of preventive effect of 5B11 on RSV type B virus in cotton rat model

In the present application, the preventive effects of 5B11 on RSV B virus were further evaluated in cotton rat model. The grouping designed in the experiment was shown in Table 6, and there were 5 groups in total, 4 to 5 cotton rats in each group. For the antibody groups, the antibody was diluted in advance with PBS to 15 mg/mL and 1.5 mg/mL for later use according to high dose (15 mg/kg) and low dose (1.5 mg/kg). On the day before challenge (Day -1), each rat was intramuscularly injected with the antibody in a certain volume according to the rat body weight into the right hind limb; 24 hours later (Day 0), 100 µL of RSV B 18537 virus with a titer of 3.8×10⁶ PFU/mL was administered the nasal cavity. For the PBS group, 100 µL of PBS was intramuscularly injected into the hind limb of each cotton rat one day before the challenge; 24 hours later, 100 µL of RSV B 18537 virus with a titer of 3.8×10⁶ PFU/mL was administered into the nasal cavity. The detection indicators were the same as the preventive experiments of 5B11 on RSV type A virus in the cotton rat model.

**Table 6: Grouping for experiment of evaluation of protective effects of RSV F protein antibodies on cotton rats**

| Group | Infection virus | Drug intervention | Number of cotton rats |
|---|---|---|---|
| PBS group | RSV B 18537 | PBS | 5 |
| 5B11 high dose group | RSV B 18537 | 5B11 (15 mg/kg) | 4 |
| 5B11 low dose group | RSV B 18537 | 5B11 (1.5 mg/kg) | 5 |
| 1129 high dose group | RSV B 18537 | 1129 (15 mg/kg) | 4 |
| 1129 low dose group | RSV B 18537 | 1129 (1.5 mg/kg) | 5 |

The lung virus plaque detection results (panel C in Fig. 8) showed that higher virus titers could be detected in the PBS group, and the high and low dose groups of 5B11 and the high and low dose groups of 1129 all had significant statistical differences as compared with the PBS group. No virus titer was detected in the lungs of the high dose and low dose groups of 5B11, suggesting that 5B11 could completely prevent RSV lung infection at low doses; only the high dose group of 1129 had no virus titer detected and the RSV lung infection was completely prevented, while a higher virus titer was detected in the low dose group of 1129 and the viral infection was reduced by 10-fold (1 log) as compared to the PBS group, indicating that it could only inhibit viral lung infection to a certain extent.

The nose virus plaque detection results (panel D in Fig. 8) showed that higher virus titers were detected in the PBS group, and the high and low dose groups of 5B11 and the high dose group of 1129 all had significant statistical differences as compared with the PBS group, in which the nasal RSV infection was completely prevented in the high dose group of 5B11, and the viral infection was reduced by 63-fold (1.8 log) in the low dose group of 5B11 as compared with the PBS group. The viral infection was reduced by 4-fold (0.6 log) in the low dose group of 1129 as compared with the PBS group, and the viral infection was reduced by 1995-fold (3.3 log) in the high dose group of 1129 as compared with the PBS group.

The pathological inflammation of the lung tissue in each group was quantified and scored according to the degree of inflammatory cell infiltration at different parts, and the differences in inflammation scores between the groups were calculated by the analysis of variance. The pathological scores of the lungs (panels F to J in Fig.9) showed that perivasculitis, pulmonary alveolitis and interstitial inflammation in the lungs of cotton rats in each group were not obvious, while the lung inflammation in the PBS group are mainly manifested as severe tracheitis and bronchitis. For the high dose group of 5B11, there were not obvious perivasculitis, tracheitis, bronchitis, pulmonary alveolitis and interstitial pneumonia. Compared with the PBS group and the low dose group of 1129, although the tracheitis was alleviated to a certain extent in the low dose group of 5B11, there was no significant statistical difference. The high dose group of 1129 was similar to the high dose group of 5B11, and also showed no obvious tracheitis or bronchitis. However, possibly due to individual differences in experimental animals, one cotton rat in each of the high dose and low dose groups of 1129 developed strong pulmonary alveolitis and interstitial pneumonia.

In summary, 5B11 at high and low doses could completely inhibit RSV A/B virus infection in the lower respiratory tract, and 5B11 at low dose could also effectively reduce RSV A/B virus infection in the upper respiratory tract, in which the virus was reduced by 500-fold and 63-fold, respectively. In addition, no obvious lung inflammation was observed in the high and low dose groups of 5B11, suggesting that 5B11 at low dose could effectively protect cotton rats from the inflammation caused by viral infection, and 5B11 can achieve the desired protective effect with one-tenth of the dose of 1129.

### Example 9: Humanized 5B11 monoclonal antibody and evaluation of neutralizing activity and reactivity thereof

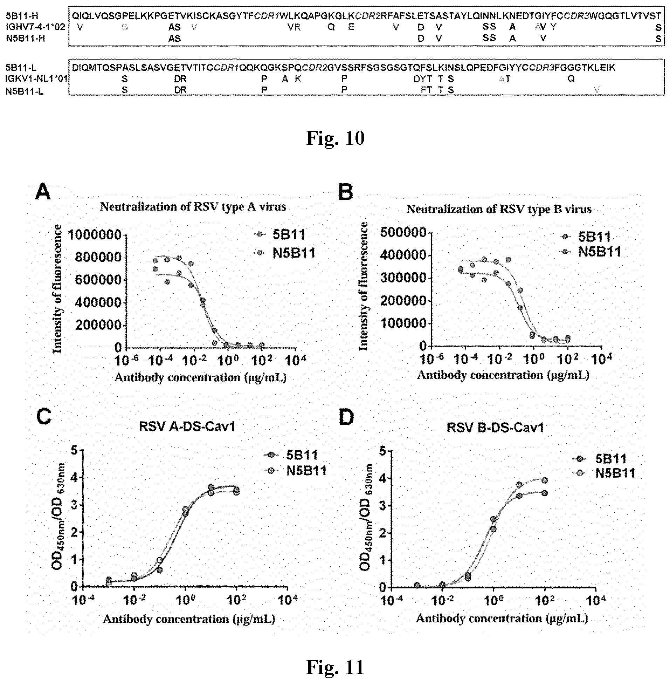

Since 5B11 was a mouse-derived antibody and had great immunogenicity, 5B11 needed to be humanized to reduce the immunogenicity of 5B11. The principles of humanization design were as follows: 1) the light and heavy chains of 5B11 antibody were aligned with the human germline gene sequences with the highest homology, respectively; 2) the amino acids in the FR that were on the CDR contact surface or were located close to the CDR were usually retained (amino acids marked in red) because these amino acids could play an important role in the binding between antigen and antibody, while the amino acids that were far away from the CDR and not hydrophobic were usually directly mutated; 3) the amino acids in the FR that were at the interface of VH-VK were usually retained (amino acids marked in blue); 4) the antibody structure was simulated to obtain exposed and internally embedded amino acids, and the internally embedded amino acids were selectively retained (amino acids marked in green) because they could affect the binding of antigen and antibody; 5) the exposed amino acids and the remaining amino acids that had little effect on antibody activity were directly mutated (amino acids marked in black). After humanization, humanized 5B11 was named as NSB11, and its humanization degree was 90% (Fig. 10). The amino acid sequences of the light chain variable region and heavy chain variable region of the humanized antibody NSB11 were set forth in SEQ ID NO: 60 and 61, respectively.

The genes of NSB11 heavy and light chain variable regions were constructed into the PTTS eukaryotic expression vector encoding the human immunoglobulin constant region stored in the laboratory to construct the plasmid PTT5-N5B 11-heavy chain (which contained the nucleotide sequence encoding the heavy chain constant region as set forth in SEQ ID NO: 62) and PTT5-NSB11-light chain (which contained the nucleotide sequence encoding the light chain constant region as set forth in SEQ ID NO: 63) respectively, the double plasmids were transiently transferred into expi-293 cells for expression, the supernatant was harvested after 7 days, protein A was used to purify the supernatant to obtain the antibody NSB11. Then, the neutralizing activity and binding activity of the purified NSB11 were detected (the methods for detecting the neutralizing activity and binding activity were referred to Example 2 and Example 3, respectively). The experimental results showed that the neutralizing activity (panels A to B in Fig.11) and binding activity (panels C to D in Fig. 11) of the humanized antibody NSB11 were consistent with those of the mouse antibody 5B11.

Although the specific embodiments of the present application have been described in detail, those skilled in the art will understand that various modifications and changes can be made to the details based on all teachings that have been disclosed, and these changes are within the scope of the present application. The full scope of the present application is given by the appended claims and any equivalents thereof.

## Claims

1. An antibody or antigen-binding fragment thereof, which specifically binds to an epitope of respiratory syncytial virus (RSV) pre-F protein, wherein the epitope comprises the amino acid residues at positions 294-295 and at least 6 amino acid residues (e.g., non-consecutive amino acid residues) within the amino acid residues at positions 160-182 of the pre-F protein;
preferably, the epitope comprises at least the amino acid residues at positions 161, 165, 166, 169, 180, 182, 294, and 295;
preferably, the epitope further comprises the amino acid residue at position 196;
preferably, the epitope further comprises the amino acid residues at positions 162 and 184;
preferably, the epitope comprises the amino acid residues at positions 161-182 (e.g., the amino acid residues at positions 161-184) and the amino acid residues at positions 294-295;
preferably, the epitope is a conformational epitope;
preferably, the position of amino acid residue is determined according to SEQ ID NO: 1;
preferably, the epitope comprises at least E161, N165, K166, S169, S180, S182, E294 and E295; preferably, the epitope further comprises K196; preferably, the epitope further comprises G162 and G184.

2. An antibody or antigen-binding fragment thereof, which comprises:
(a) a heavy chain variable region (VH) comprising the following three CDRs: a VH CDR1 comprising the sequence as set forth in SEQ ID NO: 5 or variant thereof, a VH CDR2 comprising the sequence as set forth in SEQ ID NO: 6 or variant thereof, a VH CDR3 comprising the sequence as set forth in SEQ ID NO: 7 or variant thereof; and/or,
(b) a light chain variable region (VL) comprising the following three CDRs: a VL CDR1 comprising the sequence as set forth in SEQ ID NO: 8 or variant thereof, a VL CDR2 comprising the sequence as set forth in SEQ ID NO: 9 or variant thereof, a VL CDR3 comprising the sequence as set forth in SEQ ID NO: 10 or variant thereof;
wherein, the variant has a sequence identity of at least 70%, at least 80%, at least 85%, at least 90%, at least 91%, at least 92%, at least 93%, at least 94%, at least 95% %, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
preferably, the antibody or antigen-binding fragment thereof is capable of specifically binding to RSV pre-F protein;
preferably, the antibody or antigen-binding fragment thereof competes with the antibody or antigen-binding fragment thereof according to claim 1 to bind to RSV pre-F protein;
preferably, the antibody or antigen-binding fragment thereof binds to the same epitope of RSV pre-F protein as the antibody or antigen-binding fragment thereof according to claim 1.

3. The antibody or antigen-binding fragment thereof according to claim 1 or 2, wherein:
(i) the antibody or antigen-binding fragment thereof comprises: the following three heavy chain CDRs: a VH CDR1 with the sequence as set forth in SEQ ID NO: 5, a VH CDR2 with the sequence as set forth in SEQ ID NO: 6, and a VH CDR3 with the sequence as set forth in SEQ ID NO: 7; and/or, the following three light chain CDRs: a VL CDR1 with the sequence as set forth in SEQ ID NO: 8, a VL CDR2 with the sequence as set forth in SEQ ID NO: 9, a VL CDR3 with the sequence as set forth in SEQ ID NO: 10; and/or,
(ii) the antibody or antigen-binding fragment thereof comprises: three CDRs contained in the heavy chain variable region (VH) as set forth in SEQ ID NO: 3 or 60; and/or, three CDRs contained in the light chain variable region (VL) as set forth in SEQ ID NO: 4 or 61; preferably, the three CDRs contained in the VH and/or the three CDRs contained in the VL are defined by the Kabat, IMGT or Chothia numbering system.

4. The antibody or antigen-binding fragment thereof according to any one of claims 1 to 3, wherein the antibody or antigen-binding fragment thereof comprises:
a VH comprising the sequence as set forth in SEQ ID NO: 3 or variant thereof, and/or, a VL comprising the sequence as set forth in SEQ ID NO: 4 or variant thereof;
wherein, the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or more amino acids (e.g., a substitution, deletion or addition of 1, 2, 3, 4, 5, 6, 7, 8, 9, or 10 amino acids) as compared to the sequence from which it is derived; preferably, the substitution is a conservative substitution;
preferably, the antibody or antigen-binding fragment thereof comprises: a VH as set forth in SEQ ID NO: 3, and/or a VL as set forth in SEQ ID NO: 4.

5. An antibody or antigen-binding fragment thereof, which specifically binds to an epitope of respiratory syncytial virus (RSV) pre-F protein, wherein the epitope comprises at least 5 amino acid residues (e.g., non-consecutive amino acid residues) within the amino acid residues at positions 160-185 and 290-295 of the pre-F protein;
preferably, the epitope comprises at least the amino acid residues at positions 161, 162, 184, 293 and 294;
preferably, the epitope comprises the amino acid residues at positions 161-184 and the amino acid residues at positions 293-294;
preferably, the epitope is a conformational epitope;
preferably, the position of amino acid residue is determined according to SEQ ID NO: 1;
preferably, the epitope comprises at least E161, G162, G184, K293 and E294.

6. An antibody or antigen-binding fragment thereof, which comprises:
(a) a heavy chain variable region (VH) comprising the following three CDRs: a VH CDR1 comprising the sequence as set forth in SEQ ID NO: 13 or variant thereof, a VH CDR2 comprising the sequence as set forth in SEQ ID NO: 14 or variant thereof, a VH CDR3 comprising the sequence as set forth in SEQ ID NO: 15 or variant thereof; and/or,
(b) a light chain variable region (VL) comprising the following three CDRs: a VL CDR1 comprising the sequence as set forth in SEQ ID NO: 16 or variant thereof, a VL CDR2 comprising the sequence as set forth in SEQ ID NO: 17 or variant thereof, a VL CDR3 comprising the sequence as set forth in SEQ ID NO: 18 or variant thereof;
wherein, the variant has a sequence identity of at least 70%, at least 80%, at least 85%, at least 90%, at least 91%, at least 92%, at least 93%, at least 94%, at least 95% %, at least 96%, at least 97%, at least 98%, at least 99%, or 100% as compared to the sequence from which it is derive, or the variant has a substitution, deletion or addition of one or several amino acids (e.g., a substitution, deletion or addition of 1, 2 or 3 amino acids) as compared to the sequence from which it is derive; preferably, the substitution is a conservative substitution;
preferably, the antibody or antigen-binding fragment thereof is capable of specifically binding to RSV pre-F protein;
preferably, the antibody or antigen-binding fragment thereof competes with the antibody or antigen-binding fragment thereof according to claim 5 to bind to RSV pre-F protein;
preferably, the antibody or antigen-binding fragment thereof binds to the same epitope of RSV pre-F protein as the antibody or antigen-binding fragment thereof according to claim 5.

7. The antibody or antigen-binding fragment thereof according to claim 5 or 6, wherein:
(i) the antibody or antigen-binding fragment thereof comprises: the following three heavy chain CDRs: a VH CDR1 with the sequence as set forth in SEQ ID NO: 13, a VH CDR2 with the sequence as set forth in SEQ ID NO: 14, and a VH CDR3 with the sequence as set forth in SEQ ID NO: 15; and/or, the following three light chain CDRs: a VL CDR1 with the sequence as set forth in SEQ ID NO: 16, a VL CDR2 with the sequence as set forth in SEQ ID NO: 17, and a VL CDR3 with the sequence as set forth in SEQ ID NO: 18; and/or,
(ii) the antibody or antigen-binding fragment thereof comprises: three CDRs contained in the heavy chain variable region (VH) as set forth in SEQ ID NO: 11; and/or, three CDRs contained in the light chain variable region (VL) as set forth in SEQ ID NO: 12; preferably, the three CDRs contained in the VH and/or the three CDRs contained in the VL are defined by the Kabat, IMGT or Chothia numbering system.

8. The antibody or antigen-binding fragment thereof according to any one of claims 5 to 7, wherein the antibody or antigen-binding fragment thereof comprises:
a VH comprising the sequence as set forth in SEQ ID NO: 11 or variant thereof, and/or a VL comprising the sequence as set forth in SEQ ID NO: 12 or variant thereof;
wherein, the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or more amino acids (e.g., a substitution, deletion or addition of 1, 2, 3, 4, 5, 6, 7, 8, 9, or 10 amino acids) as compared to the sequence from which it is derived; preferably, the substitution is a conservative substitution;
preferably, the antibody or antigen-binding fragment thereof comprises: a VH as set forth in SEQ ID NO: 11, and/or a VL as set forth in SEQ ID NO: 12.

9. An antibody or antigen-binding fragment thereof, which specifically binds to an epitope of respiratory syncytial virus (RSV) pre-F protein, wherein the epitope comprises at least 3 amino acid residues (e.g., non-consecutive amino acid residues) within the amino acid residues at positions 160-185 of the pre-F protein;
Preferably, the epitope comprises at least the amino acid residues at positions 161, 162, and 184;
preferably, the epitope comprises the amino acid residues at positions 161-184;
preferably, the epitope is a conformational epitope;
preferably, the position of amino acid residue is determined according to SEQ ID NO: 1;
preferably, the epitope comprises at least E161, G162 and G184.

10. An antibody or antigen-binding fragment thereof, which comprises:
(a) a heavy chain variable region (VH) comprising the following three CDRs: a VH CDR1 comprising the sequence as set forth in SEQ ID NO: 21 or variant thereof, a VH CDR2 comprising the sequence as set forth in SEQ ID NO: 22 or variant thereof, a VH CDR3 comprising the sequence as set forth in SEQ ID NO: 23 or variant thereof; and/or,
(b) a light chain variable region (VL) comprising the following three CDRs: a VL CDR1 comprising the sequence as set forth in SEQ ID NO: 24 or variant thereof, a VL CDR2 comprising the sequence as set forth in SEQ ID NO: 25 or variant thereof, a VL CDR3 comprising the sequence as set forth in SEQ ID NO: 26 or variant thereof;
wherein, the variant has a sequence identity of at least 70%, at least 80%, at least 85%, at least 90%, at least 91%, at least 92%, at least 93%, at least 94%, at least 95% %, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
preferably, the antibody or antigen-binding fragment thereof is capable of specifically binding to RSV pre-F protein.
preferably, the antibody or antigen-binding fragment thereof competes with the antibody or antigen-binding fragment thereof according to claim 9 to bind to RSV pre-F protein;
preferably, the antibody or antigen-binding fragment thereof binds to the same epitope of RSV pre-F protein as the antibody or antigen-binding fragment thereof according to claim 9.

11. The antibody or antigen-binding fragment thereof according to claim 9 or 10, wherein:
(i) the antibody or antigen-binding fragment thereof comprises: the following three heavy chain CDRs: a VH CDR1 with the sequence as set forth in SEQ ID NO: 21, a VH CDR2 with the sequence as set forth in SEQ ID NO: 22, and a VH CDR3 with the sequence as set forth in SEQ ID NO: 23; and/or, the following three light chain CDRs: a VL CDR1 with the sequence as set forth in SEQ ID NO: 24, a VL CDR2 with the sequence as set forth in SEQ ID NO: 25, and a VL CDR3 with the sequence as set forth in SEQ ID NO: 26; and/or,
(ii) the antibody or antigen-binding fragment thereof comprises: three CDRs contained in the heavy chain variable region (VH) as set forth in SEQ ID NO: 19; and/or, three CDRs contained in the light chain variable region (VL) as set forth in SEQ ID NO: 20; preferably, the three CDRs contained in the VH and/or the three CDRs contained in the VL are defined by the Kabat, IMGT or Chothia numbering system.

12. The antibody or antigen-binding fragment thereof according to any one of claims 9 to 11, wherein the antibody or antigen-binding fragment thereof comprises:
a VH comprising the sequence as set forth in SEQ ID NO: 19 or variant thereof, and/or a VL comprising the sequence as set forth in SEQ ID NO: 20 or variant thereof;
wherein the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletions or addition of one or several amino acids (e.g., a substitution, deletion or addition of 1, 2, 3, 4, 5, 6, 7, 8, 9, or 10 amino acids) as compared to the sequence from which it is derived; preferably, the substitution is a conservative substitution;
preferably, the antibody or antigen-binding fragment thereof comprises: a VH as set forth in SEQ ID NO: 19, and/or a VL as set forth in SEQ ID NO: 20.

13. The antibody or antigen-binding fragment thereof according to any one of claims 1 to 12, which is humanized;
preferably, the antibody or antigen-binding fragment thereof comprises a framework sequence derived from a human immunoglobulin;
preferably, the antibody or antigen-binding fragment thereof comprises: a heavy chain framework region sequence derived from a human heavy chain germline sequence, and a light chain framework region sequence derived from a human light chain germline sequence;
preferably, the antibody or antigen-binding fragment thereof comprises: a VH as set forth in SEQ ID NO: 60, and/or a VL as set forth in SEQ ID NO: 61.

14. The antibody or antigen-binding fragment thereof according to any one of claims 1 to 13, which further comprises a constant region derived from a murine or human immunoglobulin;
preferably, the heavy chain of the antibody or antigen-binding fragment thereof comprises a heavy chain constant region derived from a human immunoglobulin (e.g., IgG1, IgG2, IgG3 or IgG4), and the light chain of the antibody or antigen-binding fragment thereof comprises a light chain constant region derived from a human immunoglobulin (e.g., an immunoglobulin comprising κ or λ chain);
preferably, the heavy chain of the antibody or antigen-binding fragment thereof comprises a heavy chain constant region as set forth in SEQ ID NO: 62, and the light chain of the antibody or antigen-binding fragment thereof comprises a light chain constant region as set forth in SEQ ID NO: 63.

15. The antibody or antigen-binding fragment thereof according to any one of claims 1 to 14, wherein the antigen-binding fragment is selected from the group consisting of Fab, Fab', (Fab')₂, Fv, disulfide-linked Fv, scFv, diabody and single domain antibody (sdAb); and/or, the antibody is a murine antibody, a chimeric antibody, a humanized antibody, a bispecific antibody or a multispecific antibody.

16. The antibody or antigen-binding fragment thereof according to any one of claims 1 to 15, wherein the antibody or antigen-binding fragment thereof has one or more of the following characteristics:
(a) neutralizing an RSV (e.g., RSV type A and/or type B) in vitro or in a subject (e.g., a human);
(b) blocking or inhibiting the fusion of an RSV (e.g., RSV type A and/or type B) with a cell in vitro or in a subject (e.g., a human);
(c) preventing and/or treating an RSV (e.g., RSV type A and/or type B) infection or a disease associated with an RSV (e.g., RSV type A and/or type B) infection (e.g., pneumonia, such as pediatric pneumonia).

17. An isolated nucleic acid molecule, which encodes the antibody or antigen-binding fragment thereof according to any one of claims 1 to 16, or its heavy chain variable region and/or light chain variable region.

18. A vector, which comprises the nucleic acid molecule according to claim 17; preferably, the vector is a cloning vector or an expression vector.

19. A host cell, which comprises the nucleic acid molecule according to claim 17 or the vector according to claim 18.

20. A method for preparing the antibody or antigen-binding fragment thereof according to any one of claims 1 to 16, comprising culturing the host cell according to claim 19 under conditions that allow expression of the antibody or antigen-binding fragment thereof, and recovering the antibody or antigen-binding fragment thereof from a culture of the cultured host cell.

21. A pharmaceutical composition, which comprises the antibody or antigen-binding fragment thereof according to any one of claims 1 to 16, and a pharmaceutically acceptable carrier and/or excipient.

22. Use of the antibody or antigen-binding fragment thereof according to any one of claims 1 to 16 in the manufacture of a medicament, wherein the medicament is used for neutralizing the virulence of RSV, or inhibiting or blocking the fusion of RSV and cells, or preventing and/or treating an RSV infection or a disease associated with an RSV infection (e.g., pneumonia, such as pediatric pneumonia) in a subject;
preferably, the subject is a mammal, such as a human;
preferably, the antibody or antigen-binding fragment thereof is used alone or in combination with an additional pharmaceutically active agent.

23. A method for preventing and/or treating an RSV infection or a disease associated with an RSV infection (e.g., pneumonia, such as pediatric pneumonia) in a subject (e.g., a human), comprising: administering to the subject in need an effective amount of the antibody or antigen-binding fragment thereof according to any one of claims 1 to 16 or the pharmaceutical composition according to claim 21.

24. A conjugate, which comprises the antibody or antigen-binding fragment thereof according to any one of claims 1 to 16, and a detectable label connected to the antibody or antigen-binding fragment thereof;
preferably, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide and biotin.

25. A kit, which comprises the antibody or antigen-binding fragment thereof according to any one of claims 1 to 16 or the conjugate according to claim 24;
preferably, the kit comprises the conjugate according to claim 24;
preferably, the kit comprises the antibody or antigen-binding fragment thereof according to any one of claims 1 to 16, and optionally a second antibody that specifically recognizes the antibody or antigen-binding fragment thereof; optionally, the second antibody further comprises a detectable label such as an enzyme (e.g., horseradish peroxidase or alkaline phosphatase), a chemiluminescent reagent (e.g., acridinium ester, luminol and derivative thereof, or ruthenium derivative), a fluorescent dye (e.g., fluorescein or fluorescent protein), a radionuclides or a biotin.

26. A method for detecting the presence or level of RSV in a sample, which comprises using the antibody or antigen-binding fragment thereof according to any one of claims 1 to 16 or the conjugate according to claim 24;
preferably, the method is an immunological assay, such as an immunoblotting assay, an enzyme immunoassay (e.g., ELISA), a chemiluminescence immunoassay, a fluorescent immunoassay or a radioimmunoassay;
preferably, the method comprises using the conjugate according to claim 24;
preferably, the method comprises using the antibody or antigen-binding fragment thereof according to any one of claims 1 to 16, and the method further comprises using a second antibody carrying a detectable label (e.g., an enzyme (e.g., horseradish peroxidase or alkaline phosphatase), a chemiluminescent reagent (e.g., acridinium ester, luminol and derivative thereof, or ruthenium derivative), a fluorescent dye (e.g., fluorescein or fluorescent protein), a radionuclides or a biotin) to detect the antibody or antigen-binding fragment thereof;
preferably, the method comprises: (1) contacting the sample with the antibody or antigen-binding fragment thereof or the conjugate; (2) detecting the formation of an antigen-antibody immune complex or detecting an amount of the immune complex; preferably, the formation of the immune complex indicates the presence of RSV or RSV-infected cells.

27. Use of the antibody or antigen-binding fragment thereof according to any one of claims 1 to 16 or the conjugate according to claim 24 in the manufacture of a kit, wherein the kit is used for detecting the presence or level of RSV in a sample, and/or for diagnosing whether a subject is infected with RSV;
preferably, the kit detects the presence or level of RSV in the sample by the method according to claim 26;
preferably, the sample is a body fluid sample (e.g., a secretion of respiratory tract) or a tissue sample (e.g., a sample of respiratory tract tissue) from a subject (e.g., a mammal, preferably a human).
